(19) Europäisches Patentamt / European Patent Office / Office européen des brevets

(11) **EP 2 786 153 B1**

(12) **EUROPEAN PATENT SPECIFICATION**

(45) Date of publication and mention of the grant of the patent:
**09.01.2019 Bulletin 2019/02**

(51) Int Cl.:
***G01N 33/68*** *(2006.01)*     ***C12N 5/00*** *(2006.01)*

(21) Application number: **12791213.7**

(22) Date of filing: **28.11.2012**

(86) International application number:
**PCT/EP2012/073878**

(87) International publication number:
**WO 2013/079558 (06.06.2013 Gazette 2013/23)**

(54) **ISOTOPIC LABELING OF HIGHER ORGANISMS**

ISOTOPISCHE MARKIERUNG VON HÖHEREN ORGANISMEN

MARQUAGE ISOTOPIQUE D'ORGANISMES SUPÉRIEURS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.11.2011 GB 201120484**
**28.11.2011 EP 11191025**

(43) Date of publication of application:
**08.10.2014 Bulletin 2014/41**

(73) Proprietor: **Silantes GmbH**
**80339 Munich (DE)**

(72) Inventors:
• **KRÜGER, Marcus**
**61231 Bad Nauheim (DE)**
• **HEUMANN, Hermann**
**81479 München (DE)**

(74) Representative: **Huenges, Martin**
**Maiwald Patentanwalts- und Rechtsanwaltsgesellschaft mbH Elisenhof Elisenstraße 3 80335 München (DE)**

(56) References cited:
**US-A1- 2007 082 399**

• **ANN WESTMAN-BRINKMALM ET AL: "SILAC zebrafish for quantitative analysis of protein turnover and tissue regeneration", JOURNAL OF PROTEOMICS, ELSEVIER, AMSTERDAM, NL, vol. 75, no. 2, 10 August 2011 (2011-08-10), pages 425-434, XP028124870, ISSN: 1874-3919, DOI: 10.1016/J.JPROT.2011.08.008 [retrieved on 2011-08-18]**
• **M. LOOSO ET AL: "Advanced Identification of Proteins in Uncharacterized Proteomes by Pulsed in Vivo Stable Isotope Labeling-based Mass Spectrometry", MOLECULAR & CELLULAR PROTEOMICS, vol. 9, no. 6, 1 June 2010 (2010-06-01), pages 1157-1166, XP055055358, ISSN: 1535-9476, DOI: 10.1074/mcp.M900426-MCP200**
• **JULIUS FREDENS ET AL: "Quantitative proteomics by amino acid labeling in C. elegans", NATURE METHODS, vol. 8, no. 10, 1 January 2011 (2011-01-01), pages 845-847, XP055055415, ISSN: 1548-7091, DOI: 10.1038/nmeth.1675**
• **J. W. GOUW ET AL: "Quantitative Proteomics by Metabolic Labeling of Model Organisms", MOLECULAR & CELLULAR PROTEOMICS, vol. 9, no. 1, 1 January 2010 (2010-01-01), pages 11-24, XP055055406, ISSN: 1535-9476, DOI: 10.1074/mcp.R900001-MCP200**
• **TARBOUSH RANIA A ET AL: "Contribution of catabolic tissue replacement to the turnover of stable isotopes in Danio rerio", CANADIAN JOURNAL OF ZOOLOGY, vol. 84, no. 10, October 2006 (2006-10), pages 1453-1460, XP009167808, ISSN: 0008-4301**

**(Cont. next page)**

• A. KONZER ET AL: "Stable isotope labelling in zebrafish allows in vivo monitoring of cardiac morphogenesis", MOLECULAR & CELLULAR PROTEOMICS, 1 February 2013 (2013-02-01), XP055055357, ISSN: 1535-9476, DOI: 10.1074/mcp.M111.015594

Remarks:
The file contains technical information submitted after the application was filed and not included in this specification

**Description**

FIELD OF THE INVENTION

The invention generally relates to metabolic labeling of fish as a target organism with a stable isotope. In a first aspect, the present invention relates to a method for metabolic labeling of a fish with a stable isotope. The present invention also relates to a method of producing a diet for metabolic labeling of a fish and to a diet composition for metabolic labeling of a target organism. In a further aspect the invention describes the use of the diet composition according to the present invention for producing a metabolically labeled fish as a reference for a quantitative proteomic approach. The present invention also relates, in a further aspect, to the use of a diet composition for pulse SILAC labeling. The present invention also relates to the use of a fish labeled by the method according to the first aspect of the present invention as a spike-in standard in quantitative proteomics approaches.

BACKGROUND OF THE INVENTION

A systems biology approach requires quantitative information about the proteome, metabolome and lipidome. The biological system can be a eukaryotic or prokaryotic cell culture, an organism or any part of an organism such as tissues. The system may also be a mixture of eukaryotic or prokaryotic cells, *e.g.* from a common habitat.

In order to understand the response of the system towards external signals or stress, the signaling cascade in these systems has to be analyzed. The areas of application for proteomics, metabolomics, and lipidomics are basic science and applied science (biology, medicine, psychology, veterinary medicine, biotechnology). Thus, the information of systems biology provides the basis for a better understanding of cellular function and can be exploited as diagnostics tools, *e.g.* for search of new biomarker, or used for drug development, production improvement of animals, and for approaches in the area of personalized medicine. Such data can be generated using high throughput techniques such as mass spectrometry in combination with LC-techniques and/or multi-dimensional gel electrophoresis.

Over the past years, mass spectrometry based proteomics has become widely recognized to analyze complex biological samples in health and disease. Although the latest generation of mass spectrometry instrumentation implies conditions for a proteome-wide analysis the proteome-wide quantitation is still a challenging problem.

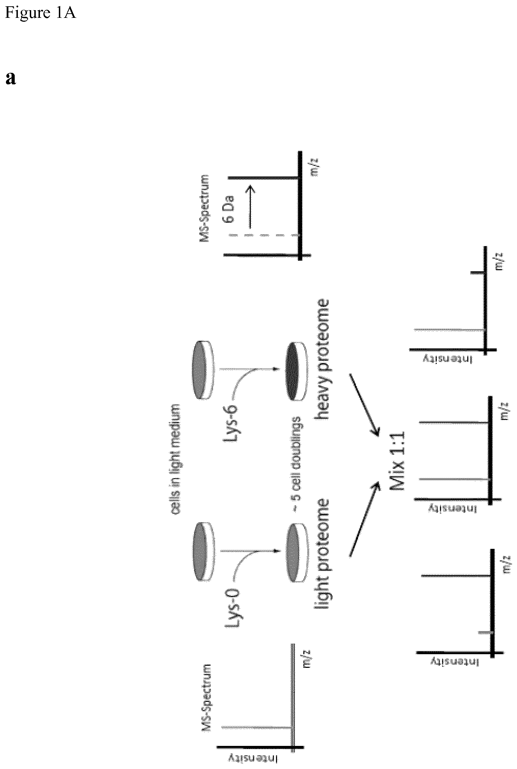

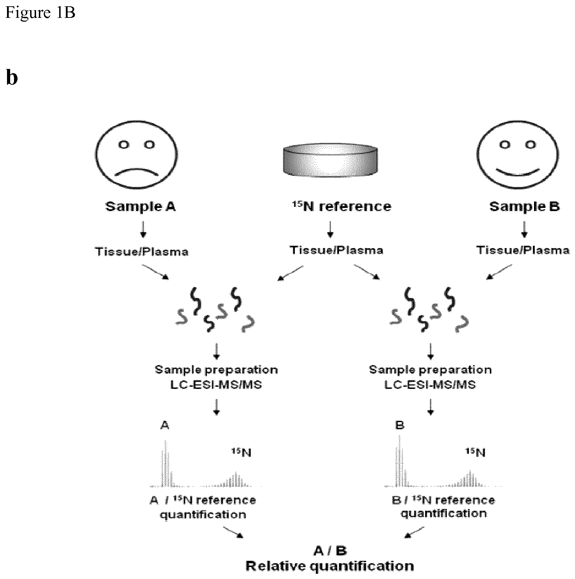

Typically, the workflow for quantification of proteins/metabolites/lipids/patterns (A) with respect to a reference proteome (B) is illustrated in Figure 1. In principle, cells of the systems (A) and (B) are mixed and the proteins, lipids, or metabolites are extracted together thus avoiding errors due to potential differences in the extraction procedure. The ability to differentiate between cells is a prerequisite for quantifying the components originating from the both systems (A) and (B). This is facilitated either by differential chemical derivatisation of the systems (A) and (B) or by using cells which are differently metabolically labeled with stable isotopes. The latter approach has thus the advantage that the cells (A) and (B) can be mixed and processed together, while chemical derivatisation requires separate disruption of the cells, and separate labeling before common extraction of the cells is possible.

Avoiding these initially separate steps in cell preparation is an important advantage of the metabolic labeling approach using stable isotopes. A high enrichment of about 98% of the stable isotopes would be advantageous for data evaluation, thus minimizing the contribution of topoisomers.

Proteome quantification by metabolic labeling with stable isotopes is a well established method described in the art (Schoenheimer, R., and Rittenberg, D. (1935); Deuterium as an Indicator in the Study of Intermediary Metabolism; Science 82, 156-157). The method could be used for analysis of metabolic pathways and turnover rates (Waterlow, J.C. (1970); Total protein turnover in animals and man; Nutr Rev 28, 115-118). The approach has recently become very popular especially for quantitative proteome analysis (Ong & Mann (2002); Stable isotope labeling by amino acids in cell culture, SILAC, as a simple and accurate approach to expression proteomics; Mol Cell Proteomics 1, 376-386; Westman-Brinkmalm, A. et al. (2011); SILAC zebrafish for quantitative analysis of protein turnover and tissue regeneration; J Proteomics 75, 425-434).

A prerequisite for this application is a high isotope enrichment (more than 95%) of the labeled cells. In order to quantify a proteome (B) relative to a proteome (A) the proteomes need to be distinguished, which can be achieved by stable isotopic labeling. In principle, two different metabolic labeling approaches using stable isotopes are known in the art, namely (i) the **SILAC** approach (**S**table **I**sotopic **L**abeling with **A**mino acids in **C**ell culture) (Ong, S.E., and Mann, M. (2007); Stable isotope labeling by amino acids in cell culture for quantitative proteomics; Methods Mol Biol 359, 37-52.) and (ii) the **SILAM** approach (**S**table **I**sotope **L**abeling of **M**ammals) with a specific isotope, mostly using $^{15}N$ for proteins and $^{13}C$ for lipids and metabolites.

In the SILAC approach, labeling of eukaryotic cells is achieved by using a synthetic cell growth media lacking the amino acids to be labeled. The cell growth media are supplemented with the lacking amino acid which either a labeled amino acid with a stable isotope to obtain the "heavy cell culture" or an unlabelled amino acid yielding the "light cell culture".

The principle of the SILAC labeling as described in Ong & Mann (2007) is illustrated in Figure 1A. Typically, a system (B: heavy) is labeled metabolically with one or two heavy amino acids. Quantification of proteins of system (A: light) versus (B: heavy) occurs by mass spectrometry. A subsequent tryptic digestion of the proteomes (A) and (B) reveals pairs of peptides, which differ in the molecular weight of their terminal light and heavy amino acids, e.g. lysines and arginines, respectively.

Although SILAC labeling was initially developed for cell culture systems several approaches in the past years extended recently the labeling procedures to living organisms such as bacteria (Soufi, B. et al. Stable isotope labeling by amino acids in cell culture (SILAC) applied to quantitative proteomics of Bacillus subtilis. J Proteome Res 9, 3638-3646).yeast (Gruhler, A. et al.; Quantitative phosphoproteomics applied to the yeast pheromone signaling pathway; Mol Cell Proteomics 4, 310-327.), worms (Tops, B.B. et al. (2010). Worms from Venus and Mars: proteomics profiling of sexual differences in Caenorhabditis elegans using in vivo 15 N isotope labeling. J Proteome Res. 9, 341.51), flies (Sury, M.D., et al. The SILAC fly allows for accurate protein quantification in vivo. Mol Cell Proteomics 9, 2173-2183), and rodents (McClatchy, D.B. et al. (2007); 15N metabolic labeling of mammalian tissue with slow protein turnover; J Proteome Res 6, 2005-2010).

Another variant of SILAC labeling is based on the use of labeled organisms as feed. Specific lysine and arginine labeling of these food organisms is achieved by using lysine and arginine auxotrophic strains (Gruhler, A. et al.; Quantitative phosphoproteomics applied to the yeast pheromone signaling pathway; Mol Cell Proteomics 4, 310-327.)

An alternative approach is termed the SILAM labeling or global uniform labeling, which is schematically depicted in Figure 1B. Typically, global labeling of prokaryotic and eukaryotic cells is facilitated by synthetic media in which the compounds containing N- and C-atoms, respectively, are replaced by the corresponding heavy isotope-labeled compound ($^{13}C$ and or $^{15}N$). Typically, in global uniform labeling by SILAM, a system (B) is completely and uniformly labeled. The work flow and quantification of proteome (A) (light) versus proteome (B) (heavy) is identical as for SILAC labeling. The major difference to SILAC labeling however is that after tryptic digestion the light and heavy peptide pairs do not differ by a constant mass difference as in the case of SILAC (McClatchy, D.B. et al. (2007); 15N metabolic labeling of mammalian tissue with slow protein turnover; J Proteome Res 6, 2005-2010). The light/heavy peptide mass differences depend on the number of stable isotopic atoms in the individual peptides sequences.

Identification of the light and the corresponding heavy peptides by mass spectrometry (MS) requires sequence data for calculating the amino acid content which in turn allows the calculation of the number of N- and C atoms of the peptides. From these data the molecular mass of the heavy peptide is predicted, facilitating determination of the mass ratios of labeled and unlabeled peptides.

Although metabolic labeling was extended to several model organisms including yeast and mouse, the extension of metabolic labeling technology to other important model organisms such as fish, amphibian or dog models can be troublesome. The development of suitable synthetic media that can be advantageously used for stable isotopic labeling of higher organisms, in which either a specific amino acid or a specific atom is completely or at least to a large extent replaced by the heavy isotopic counterpart, is very complex and complicated, since both, namely dietary as well as labeling requirements have to be fulfilled. So far, only amino acid based synthetic feed (heavy diet) for SILAC and SILAM labeling of mouse is commercially available (Silantes GmbH, Munich, CIL, Andover).

An important drawback of direct labeling via synthetic feed however is that the development of suitable synthetic media requires laborious nutritional analyses, in particular, the identification of those components that are essential for a feed. The essential components further needs to be quantified in order to obtain a balanced diet. Finally, the amino acid/atoms to be labeled in the synthetic media must be depleted and subsequently supplemented with the stable isotope label. These tasks are very tedious, if not impossible, mainly because a large number of parameters have to be considered and optimized.

Further, a minimum requirement in comparative proteomics is that the stable isotope labeling is complete or has at least a high degree of enrichment of the stable isotope. Using the prior methodology it is however difficult if not impossible to obtain a complete labeling in a complex biological system such as higher organism.

There is thus a strong need for novel diet compositions for use in the labeling of higher organisms. There is also a demand for novel labeling techniques for interesting model organisms such as, for example, zebrafish or dog for obtaining quantitative data on their proteome, metabolome or lipidome.

Hence, the state of the art lacks principle means and methods capable of efficiently metabolically labeling higher organisms with stable isotope. It is desirable to provide a labeling method that results in labeled organisms that are suitable for use as a reference in large scale quantitative proteomics approaches. This means, the isotopic enrichment (or the degree of labeling) of the reference organisms has to be high.

Hence, there is a need for novel labeling strategies leading to a high enrichment of the isotopic label in the target organism. There is a further need for such methods and means that are applicable for both amino specific labeling as well as for global uniform labeling.

OBJECTS AND SUMMARY OF THE INVENTION

In one aspect a method for metabolic labeling of a target organism with a stable isotope is provided, wherein the stable isotope is transferred from a label source to the target organism

> a) via one or more food chains, each food chain comprising of one or more cascades of ancestor organisms, wherein each cascade forms the ancestor organism for a successor organism of the following cascade, and wherein at least one food chain comprises

>> i) a straight food chain comprising at least two cascades, wherein at least two ancestor organisms of the at least two cascades are connected in series; or
>> ii) a branched food chain, wherein at least two ancestor organisms of the same or of different cascades are arranged in parallel; or

> b) via one or more one food chains of a) in combination with at least one synthetic diet labeled with the stable isotope as direct label source;
> wherein the target organism is fish;
> wherein the target organism is fed with a diet composition comprising material, cells and/or tissues derived from one or more labeled ancestor organisms of said one or more food chains or a combination thereof; wherein optionally the diet composition further comprises at least one synthetic diet labeled with the stable isotope; and wherein the stable isotope used is $^{13}$C, $^{15}$N, and/or $^2$H, or a combination thereof.

Envisaged by the present invention is a suitable strategy to metabolically label fishes. The envisaged labeling approach according to the present invention is based on the recognition, development and production of a suitable feed for the labeling of fishes. The inventors of the present invention have recognized that the isotopic label can be advantageously transferred from a source to the organism to be labeled via ancestor organisms of a food chain and have developed the concept of a stable isotope (SI) food chain.

The present invention describes a food chain for the metabolic labeling of a fish. Also envisaged by the present disclosure are labeled cell cultures which alone or in combination can be used as alternative sources of isotope label.

The labeling approach according to the present invention has several advantages over the prior art. The SI food chain based compositions according to the present invention closer resemble the physiological situation of the targeted organism in its habitat, facilitating the preparation of a biologically competent feed. The analytical effort is restricted to the analysis of the stable isotopic enrichment of the ancestors. Further, the risk of isotopic dilution is largely minimized if the transfer of the label occurs via a food chain. A further advantage over the art is that the metabolic labeling of the present invention describes how different sources having the isotopic label can be combined in order to obtain a suitable and balanced feed composition or ancestor organism for labeling of a target organism effectively.

In particular, the examples of the present application demonstrate that the method as described herein using a diet composition according to the present invention is suitable to label living zebrafish. The labeling approach according to the present invention thus allows for highly reproducible quantitation of several thousand proteins by straightforward spike in a SILAC experiment. The administration of heavy amino acids via the diet composition according to the present invention provides the basis for obtaining target organisms labeled with one or more stable isotopes suitable for a straightforward mass spectrometric-based approach to investigate tissue regeneration and turnover under complex *in vivo* conditions.

In yet another preferred embodiment of the present invention the metabolic labeling is an amino acid specific labeling.

In amino acid specific labeling, the label source may be one or more labeled amino acids.

In yet another preferred embodiment of the present invention the labeled amino acid is lysine and/or arginine.

In a preferred embodiment of the present invention the amino acid is $^{13}$C-labeled lysine.

In a preferred embodiment of the present invention the metabolic labeling is global uniform labeling. In global uniform labeling, the label source may be a labeled amino acid mixture or a labeled protein hydrolysate.

In a yet preferred embodiment of the present invention the label source may be a labeled protein hydrolysate.

In another preferred embodiment of the present invention the enrichment of the stable isotope in the target organism is at least about 90%.

In another preferred embodiment of the present invention the enrichment of the stable isotope in the target organism is at least 91%, preferably 92%, 93%, or 94%, more preferably at least about 95%, and most preferably at least about 98%.

In yet another preferred embodiment of the present invention the target organism is selected from the group consisting of fishes such as *D. rerio* (Zebrafish) or salmon.

In a further preferred embodiment of the present invention the ancestor organism of the one or more food chains is prokaryotic or eukaryotic organism such as an animal, a plant or a fungus.

In yet further preferred embodiment of the present invention the prokaryotic organism is a cyanobacterium.

In a preferred embodiment of the present invention the cyanobacterium is selected from the group consisting of *Synecococcus* and *Spirulina.*

In another preferred embodiment of the present invention the eukaryotic organism is a unicellular or multi-cellular plant.

In yet another preferred embodiment of the present invention the plant cell is an algae selected from the group consisting of *Chlamydomonas, Scenedesmus* and *Chlorella.*

In a preferred embodiment of the present invention the direct ancestor organism of the at least one food chain used for transferring the label to said target organism has an average SILAC label of at least 40%.

In further preferred embodiments of the present invention the direct ancestor organism of the at least one food chain used for transferring the label to said target organism has an average SILAC label of at least 50%, preferably of at least 60% or 70%, more preferably of at least 80%, even more preferably of at least 85%, and most preferably of at least 90%.

In another preferred embodiment of the present invention the direct ancestor organism of said at least one food chain used for transferring the label to said target organism has an average global uniform label of at least 40%.

In further preferred embodiments of the present invention the direct ancestor organism of said at least one food chain used for transferring the label to said target organism has an average global uniform label of at least 50%, preferably of at least 60% or 70%, more preferably of at least 80%, even more preferably of at least 85%, and most preferably of at least 90%.

In a second aspect, the present invention discloses a method of producing a diet for metabolic labeling of a target organism with a stable isotope, comprising

i) producing one or more ancestor organisms of a straight food chain comprising at least two cascades, wherein the one or more ancestor organisms are labeled with a stable isotope, and wherein at least two labeled ancestor organisms are connected in series, and wherein at least one labeled ancestor organism is an organism of at least the second or higher cascade; and/or

ii) producing at least two ancestor organisms of a branched food chain, wherein the at least two ancestor organisms are each labeled with a stable isotope and wherein the at least two labeled ancestor organisms of the same or of different cascades are arranged in parallel; and

iii) processing said one or more labeled ancestor organisms obtained in i) and/or ii) to obtain a diet for feeding the target organism;

wherein the target organism is fish;

wherein the target organism is fed with a diet composition comprising material, cells and/or tissues derived from one or more labeled ancestor organisms of said one or more food chains or a combination thereof; wherein optionally the diet composition further comprises at least one synthetic diet labeled with the stable isotope; and wherein the stable isotope used is $^{13}$C, $^{15}$N, and/or $^{2}$H, or a combination thereof.

In a preferred embodiment of the present invention the metabolic labeling is an amino acid specific labeling.

In yet another preferred embodiment of the present invention the ancestor organism of the lowest cascade is an auxotroph.

In a preferred embodiment of the present invention the metabolic labeling is a global uniform labeling by a labeled amino acid mixture or a labeled protein hydrolysate.

In yet a further preferred embodiment of the present invention the metabolic labeling is a global uniform labeling. In global umiform labeling, the label source may be a labeled protein hydrolysate.

In a preferred embodiment of the present invention the method of producing a diet for metabolic labeling comprises adding a synthetic diet comprising the stable isotope label as an additional label source.

In yet another preferred embodiment of the present invention a balanced diet comprises cells and/or tissues derived from one or more labeled ancestor organisms of one or more food chains and/or cells from one or more cell cultures.

In a further preferred embodiment of the present invention a balanced diet comprises a combination of two or more ancestor organisms of different cascades.

In yet another preferred embodiment of the present invention the ancestor organism of the lowest cascade is fuelled with isotope label using a synthetic medium.

In a further preferred embodiment of the present invention the target organism is *D. rerio* (Zebrafish) or an economically important fish.

In a further aspect, the present invention describes a diet composition for metabolic labeling of a target organism obtainable by the method according to the second aspect of the present invention as described above.

In yet a further aspect, the present invention relates to a diet composition for metabolic labeling of a target organism with a stable isotope, wherein the diet composition comprises material, cells, and/or tissues of

i) at least one ancestor organism of at least the second cascade of a straight food comprising at least two cascades, wherein the at least one ancestor organism is labeled with a stable isotope and wherein at least two labeled ancestor organisms are connected in series;

ii) at least two ancestor organisms of a branched food chain, wherein the at least two ancestor organisms are each labeled with a stable isotope and wherein said at least two labeled ancestor organisms of the same or of different cascades are arranged in parallel; and wherein the target organism is fish;

wherein the target organism is fed with a diet composition comprising material, cells and/or tissues derived from one or more labeled ancestor organisms of said one or more food chains or a combination thereof; wherein optionally the diet composition further comprises at least one synthetic diet labeled with the stable isotope; and wherein the stable isotope used is $^{13}$C, $^{15}$N, and/or $^{2}$H, or a combination thereof.

In a preferred embodiment of this aspect the diet composition further comprises at least one synthetic diet labeled with the stable isotope.

Another aspect of the invention is the use of the diet composition according to the present invention for producing a metabolically labeled target organism as a reference for a quantitative proteomic approach.

A further aspect of the present invention is the use of the diet composition according to the present invention for pulse SILAC labeling.

Another aspect of the invention is the use of a target organism labeled by the method of metabolic labeling according to the present invention as a spike-in standard in quantitative proteomics approaches.

## BRIEF DESCRIPTION OF THE DRAWINGS

The following drawings are illustrative of the embodiments of the invention and are not meant to limit the scope of the invention as encompassed by the claims.

**Figure 1A: Workflow for an amino acid specific SILAC proteome analysis.**
Cells of the light proteome (A) and cells of the heavy proteome (B) are mixed. The proteins are extracted and cleaved into peptides using trypsin or endoproteinase LysC. Peptides are identified by tandem mass spectrometry. Those peptides originating from the heavy proteome have a 6 Da higher mass than their light counterpart. Peptides are quantified by determining the ratio from mass spectra peaks of the light and heavy labeled peptides. (Lys-0: unlabelled lysine; Lys-6: 13C-lysine.)

**Figure 1B: SILAM approach for quantitative proteomics comparisons.**
Heavy cells (reference) are used for "spike-in" into the two cell cultures to be compared, namely cell culture (A) and cell culture (B). Depicted is a work flow of the "spiking in approach" for $^{15}$N-labeling. $^{15}$N-labelled reference material (light grey lines) is used for pair wise comparisons with samples from the two sample groups A and B (dark grey lines).

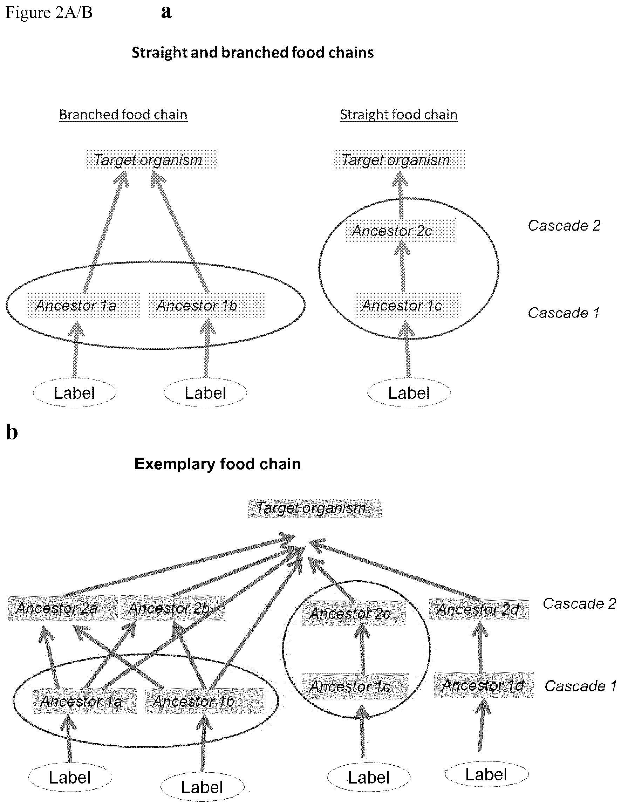

**Figure 2: The concept of SI food chain labeling**

**Figure 2A: Straight and branched food chains**
The diagram exemplary shows two types of SI food chains. Each food chain may comprise one or more cascades of ancestor organisms, wherein each cascade of a food chain forms the ancestor organism for a successor organism of the following cascade. The label fuelling a food chain is transferred from a label source via one or more ancestor organisms in the food chain to a target organism. In a straight food chain the isotope label is transferred linearly (or serially) via at least two ancestors (Ancestor 1c and 2c) of at two consecutive cascades (Cascade 1 and 2) to the target organism. A branched food chain as depicted on the left panel requires that at least two ancestor (Ancestor 1a and 1b) being arranged in parallel, or next to each other. The parallel ancestor organisms may be from the same cascade or from two different cascades.

**Figure 2B: Exemplary food chain**
Examples of numerous possibilities of food chains and food chain combinations are shown. At least one food chain is either a branched food chain or a straight food chain as indicated by the circles.

**Figure 3: Specific Examples of food chains fuelled by $^{13}$C-lysine**

**Figure 3A: Development of feed for zebrafish using the food chain concept**

Depicted is an exemplary food chain for the labeling of zebrafish (*D. rerio*). The ancestors of the lowest cascade (Cascade 1) are fuelled by $^{13}$C-lysine as label using lysine-auxotrophic strains of yeast (*S. cerevisiae*) and *E. coli.* These primary ancestor organisms (yeast, *E. coli*) subsequently serve as a feed for the organisms of the following cascades (successor organisms). The ancestor organisms (Artemia, Daphnia, *C. elegeans* and *Drosophila*) of the second cascade themselves represent the direct ancestor and may serve as a feed for the target organism. Each of the food chains as indicated by the successive arrows constitutes a straight food chain.

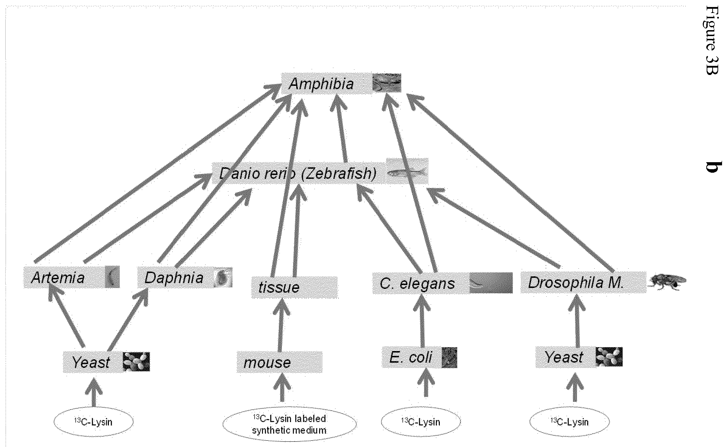

**Figure 3B: Development of feed for amphibians using the food chain concept**

Depicted is an exemplary food chain for the labeling of an amphibian. In the natural food chain, amphibians are located one cascade higher than zebrafish. The exemplary feed compositions are based on a further feed source using tissue from a mouse labeled with synthetic $^{13}$C-lysine medium for fueling of a food chain leading to metabolic labeling of the amphibian directly or by virtue of an additional cascade, *i.e.* via the zebrafish.

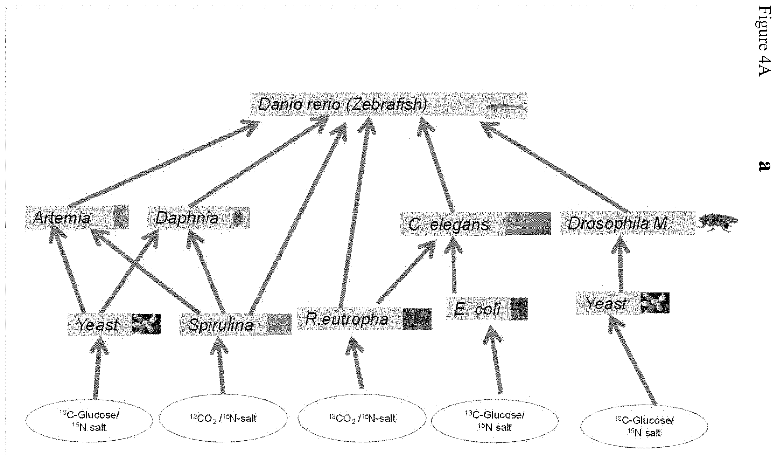

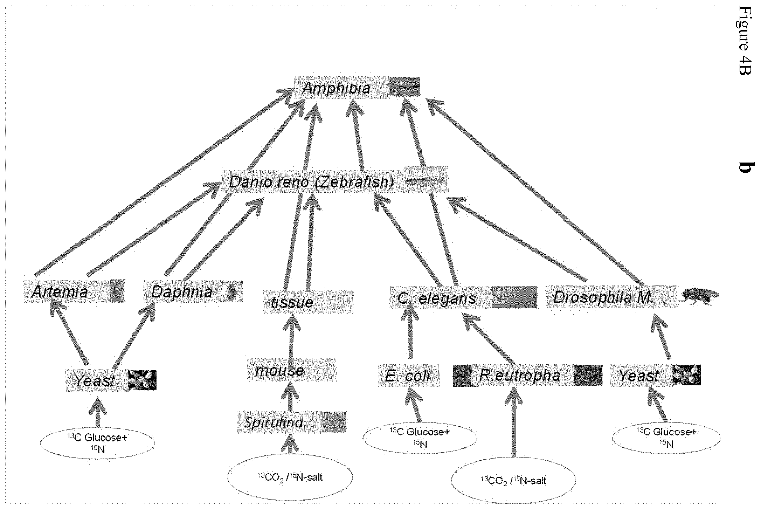

**Figure 4: Development of globally labeled zebrafish based on the SI food chain concept**

The global stable isotope food chains can be organized similar to SILAC food chains. As can be seen in the Figure, one difference is that the ancestor organisms of the lowest cascade are wild type and not auxotrophic organisms. As shown in the diagrams, this allows the inclusion of plants, algae and other organisms for which no auxotrophic organisms are available.

**Figure 4A: Global food chain labeling of zebrafish**

For global stable isotope food chain labeling also non-auxotrophic organisms such as algae (*Spirulina platensis* or *Ralstonia eutropha*) can be used. The organisms of the lowest cascade are able to grow on synthetic media containing $^{13}$C-Glucose, $^{15}$N-salt, or $^{13}$CO$_2$ as a source for the isotope label.

**Figure 4B: Global food chain labeling of an amphibian**

Organisms of different cascades may be used as direct ancestors organism for producing a suitable feed for amphibians. The SI food chains may incorporate a variety of ancestor organisms such algae (*Spirulina platensis* or *Ralstonia eutropha*) and also mouse tissue, which is not an ancestor of the natural food chain.

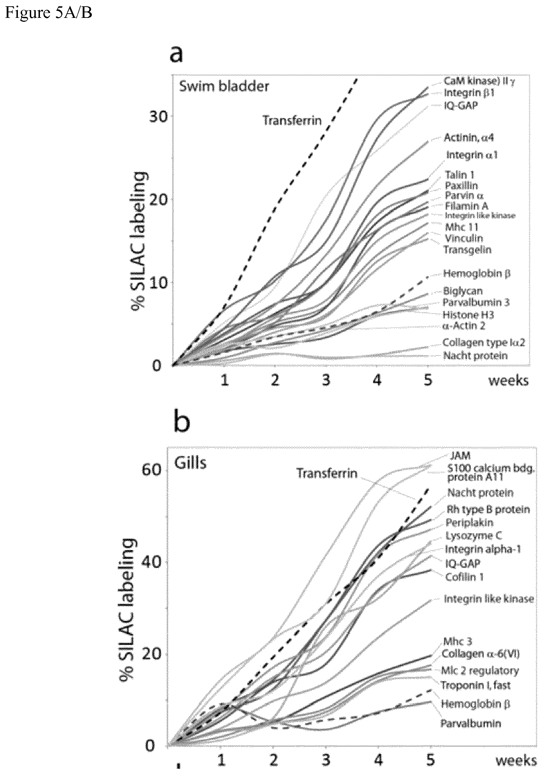

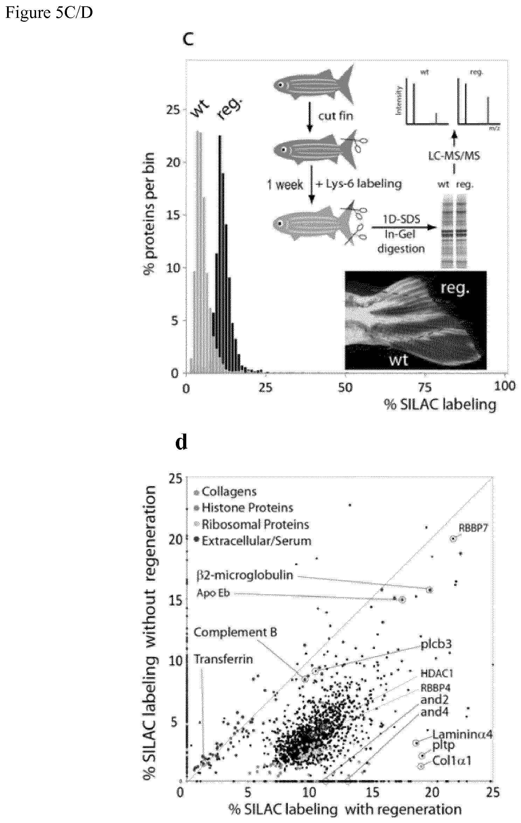

**Figure 5: Incorporation of Lys-6 and fin regeneration in *Danio rerio.***

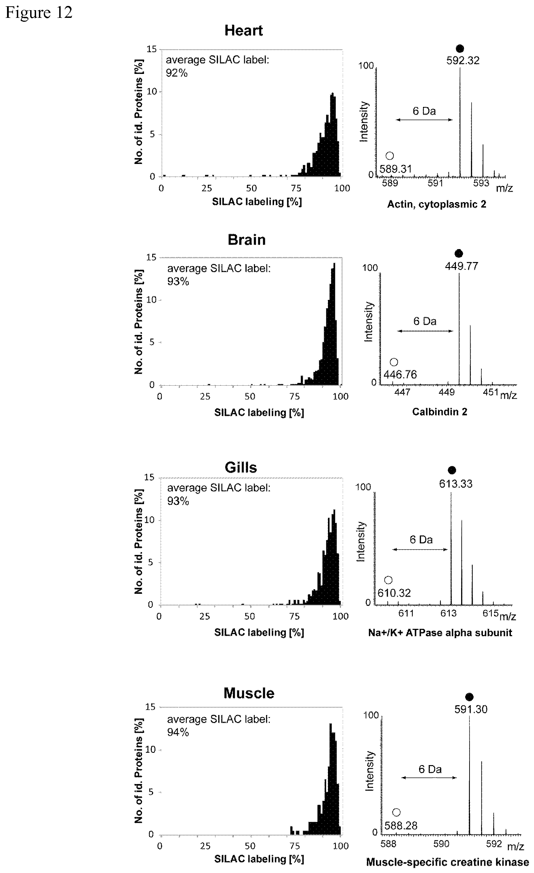

Adult wild type zebrafish were fed with the Lys-6 containing diet for a time course of 5 weeks. Each week, one zebrafish was isolated to monitor the Lys-6 incorporation in (a) swim bladder and (b) gills. Other organs are shown in Fig. 12. Percentaged incorporation of Lys-6 was calculated based on SILAC-protein ratios and plotted against the time in weeks. Transferrin and Hemoglobin are shown as dashed curves representing proteins with high (black) and low (red) Lys-6 incorporation. (c) The panel shows the relative number of proteins with specified SILAC labeling for non-regenerating (wt, grey bars) and regenerating fins (reg., black bars). The inlets in (c) illustrate the workflow for the regeneration assay. After 1 week Lys-6 administration, fins were cut as indicated by the red dashed line and processed for mass spectrometric analysis. (d) Percentaged SILAC ratios of non-regenerated fins and regenerated fins were plotted against each other. Proteins from different cellular compartments were indicated by collared dots. Diagonal line represents a 1:1 incorporation as observed for most of the secreted blood proteins (red dots). Abbreviations: Apo Eb Apolipoprotein Eb; plcb3 Phospholipase C b3; RBBP4/7 Histone binding protein 4/7; and2/4 Actinodin 2/4; HDAC1 Histone deacetylase 1; pltp Phospholipid transferprotein; Col1$\alpha$1 Collagen 1$\alpha$1.

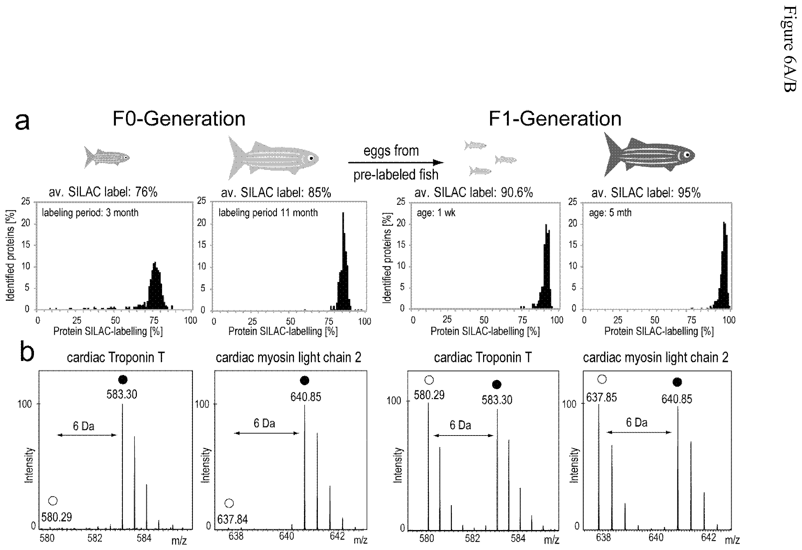

**Figure 6: Accomplished SILAC labeling of the F1 generation**

(a) Non-labeled adult (age 2 month) wild type zebrafish were fed with Lys-6 diet for 3 and 9 month. Incorporation rates were plotted against relative number of identified proteins. The analysis contains ~200 proteins per plot. The average of SILAC incorporation rates are indicated and an incorporation rate of 95% were observed after 5 month of the F1 generation (a, right histogram). (b) Selected MS-spectra of cardiac proteins detected in labeled heart tissue (left panel) confirm virtually complete Lys-6 incorporation. The right panels show a 1:1 mixing ratio of two selected peptides from non-labeled and lys-6 labeled heart tissue.

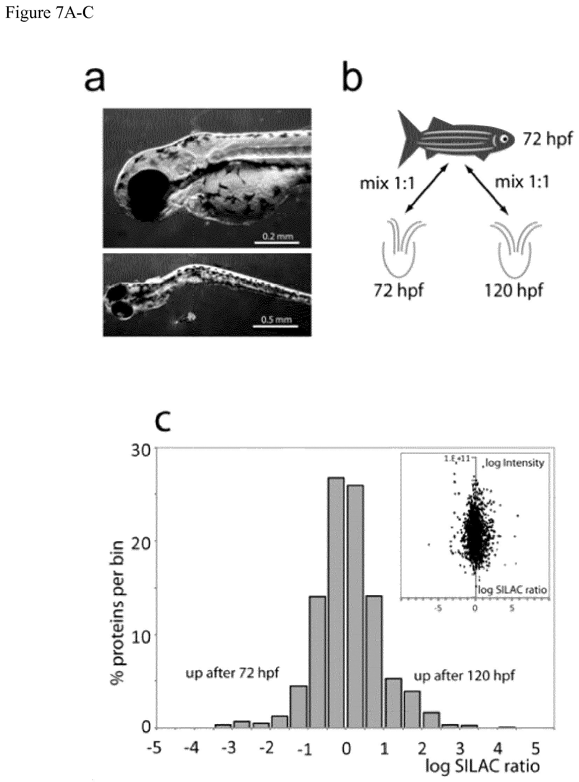

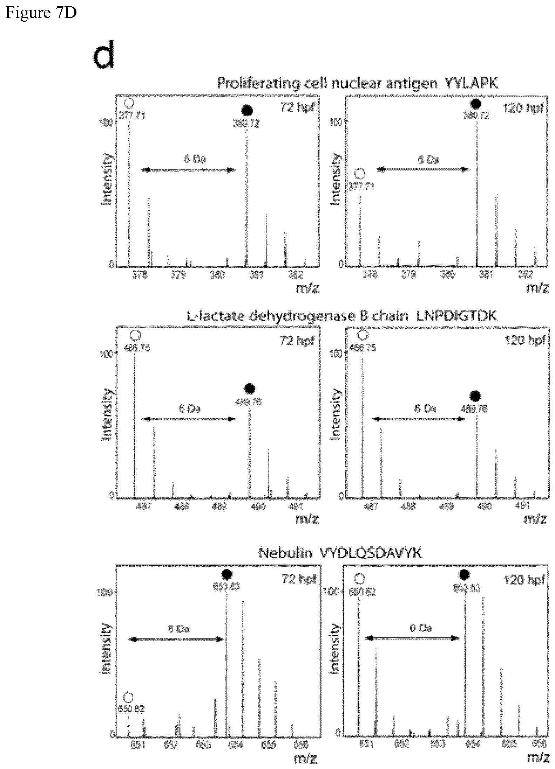

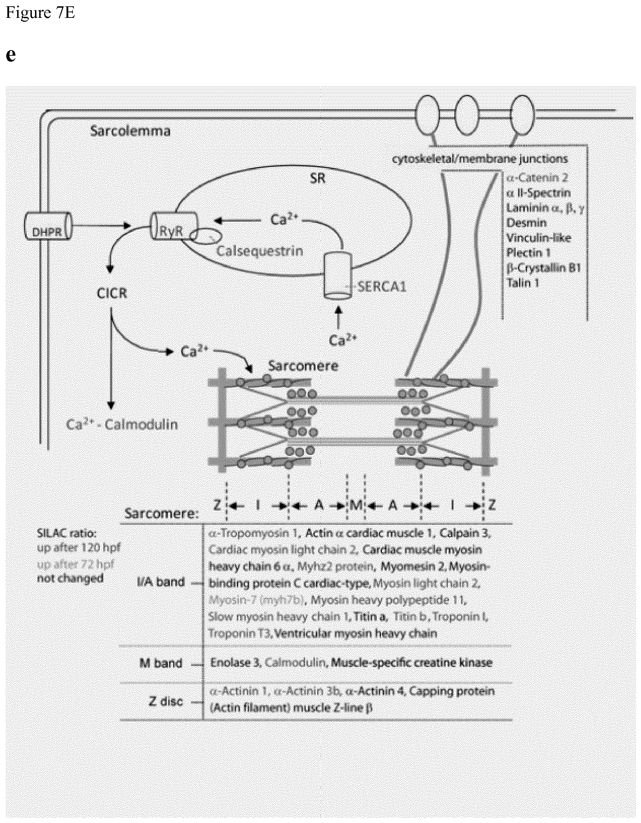

**Figure 7: Analysis of cardiac development in zebrafish.**

(a) Embryonic hearts were isolated at 72 and 120 hpf from cmlc2-GFP reporter zebrafish strain. A single isolated green heart is shown in the lower picture. (b) Lys-6 labeled tissue from 72 hpf embryos were used for a spike-in experiment to quantify hearts from two non-labeled developmental stages. (c) Histogramm of ~ 1400 quantified proteins between non-labeled 72 and 120hpf embryos. Inlet in (c) shows direct ratios from labeled embryos of selected non-labeled hearts plotted against peak intensities. Of these 172 proteins which are up-regulated ($\geq$ 2 fold

change) at 120 hpf and 86 proteins were enriched at 72 hpf. (d) Selected MS-spectra of proteins enriched at 72 hpf (PCNA), with no regulation (L-lactate dehydrogenase), and with higher abundance at 120 hpf (Nebulin). (e) Pathway analysis shows for several proteins an up-regulation at 120 hpf which are related to muscle contraction and Calcium-homeostasis.

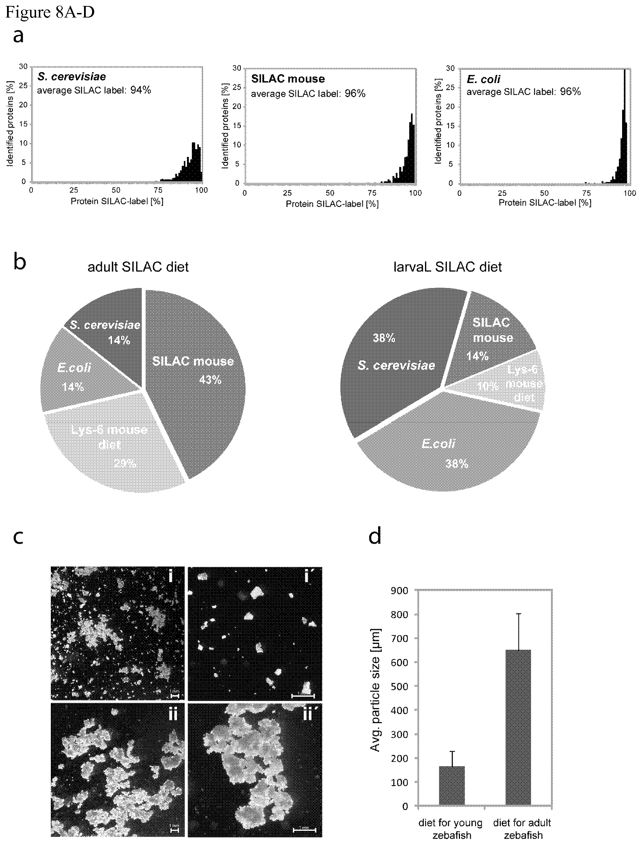

**Figure 8: Composition of the heavy diet.**
(a) The SILAC diet for the zebrafish comprises of heavy labeled cells of *E. coli, S. cerevisiae*, mouse tissue and SILAC mouse diet. (b) The SILAC diet differs in their composition according to developmental stages. (c) Shape and particle size of the diet. (d) Histogram shows average size of particles.

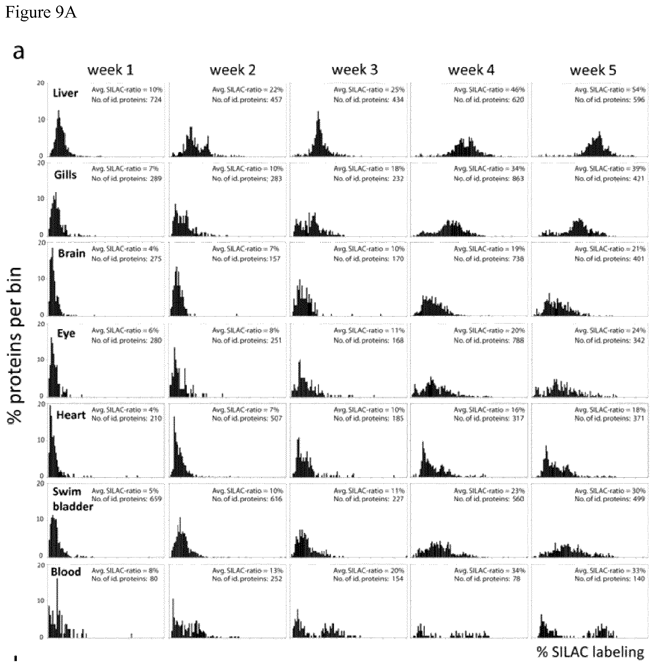

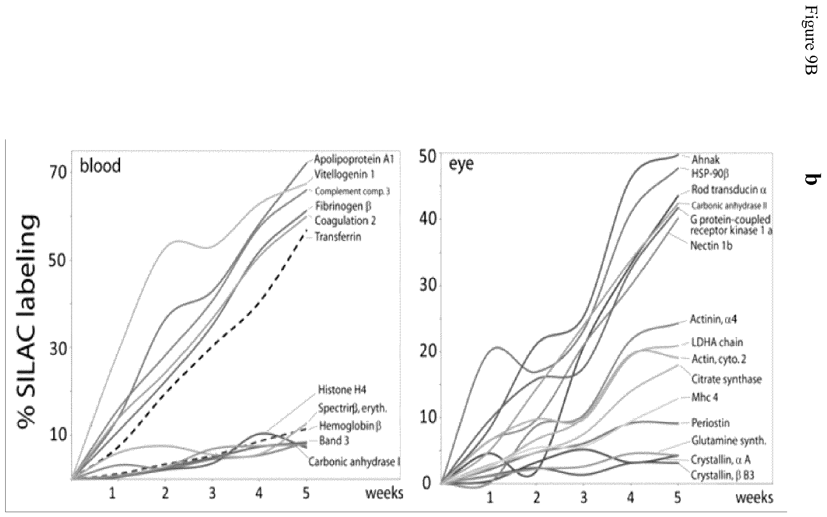

**Figure 9: Incorporation of Lys-6 of diverse zebrafish tissue.**
(a) Zebrafish is labeled by daily administration of SILAC diet and tissues were isolated over a time course of 5 weeks. The panels show relative numbers of proteins with specified Lys-6 incorporation rates. Numbers of identified proteins and average labeling are indicated in each panel. (b) Time course of selected proteins from blood and eye.

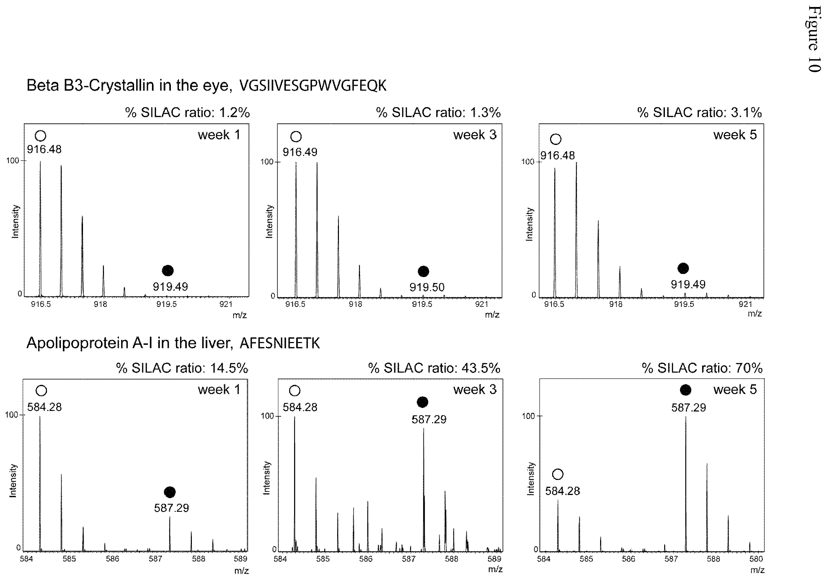

**Figure 10: Proteins with low and high turnover.**
Selected MS-spectra of peptides derived from beta-B3-Crystallin and Apolipoprotein A-I after 1 week, 3 weeks, and 5 weeks Lys-6 administration.

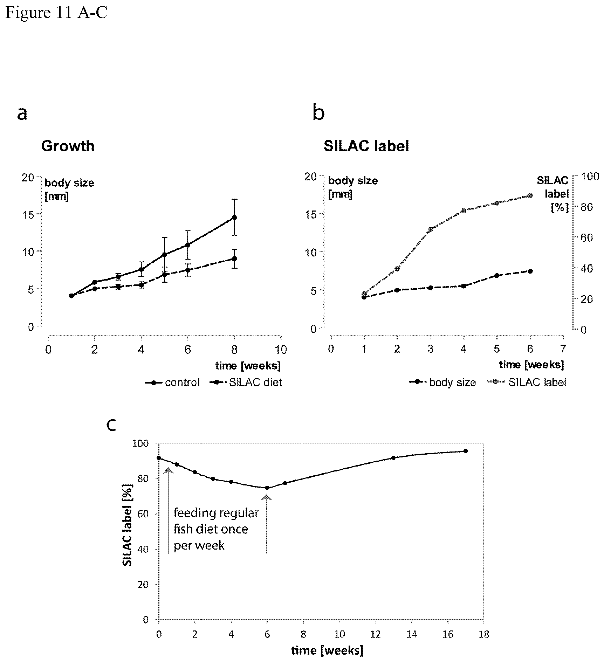

**Figure 11: Growth and fertility of the SILAC zebrafish.**
a) Growth of zebrafish embryos fed with regular diet (Special Diets Services, black curve) and SILAC diet (dashed curve) over a time course of 8 weeks after fertilization (wpf). At each time point represents 10 embryos (n=10). As indicated the average body size of SILAC embryos ranges from 9mm +/- 2mm compared to embryos administrated with regular diet (14.6 mm +/- 3mm). (b) Lys-6 incorporation increased along with body size. The upper curve represents the SILAC label. The lower curve corresponds to the body size. (c) Administration of SILAC diet supplemented with regular non-labeled food. The graph shows the % SILAC labeling plotted against the time in weeks. Arrows indicates time period with non-labeled diet.

**Figure 12: Tissue-specific Lys6-incorporation of fully labeled zebrafish.**
Several organs of adult zebrafish from the F1 generation. Relative numbers of proteins with specific Lys-6 incorporation rates are shown for heart (n=424), brain (n=729), gills (n=640) and muscle (n=199) next to exemplary MS-spectra.

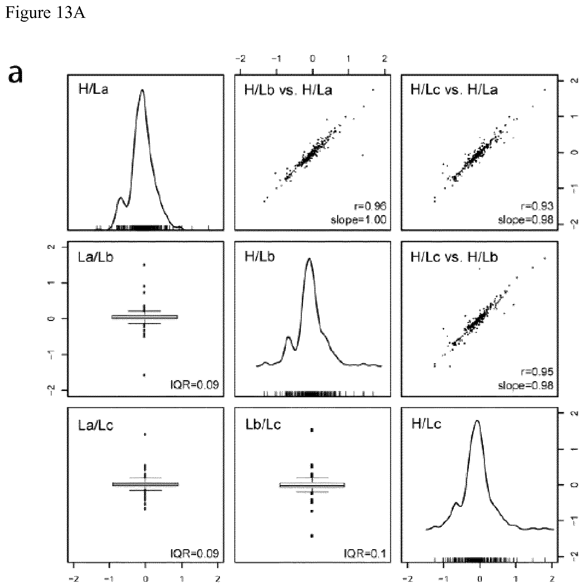

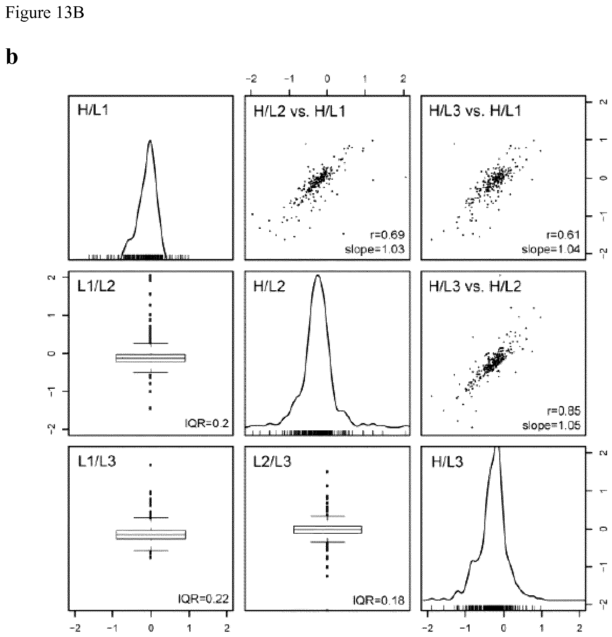

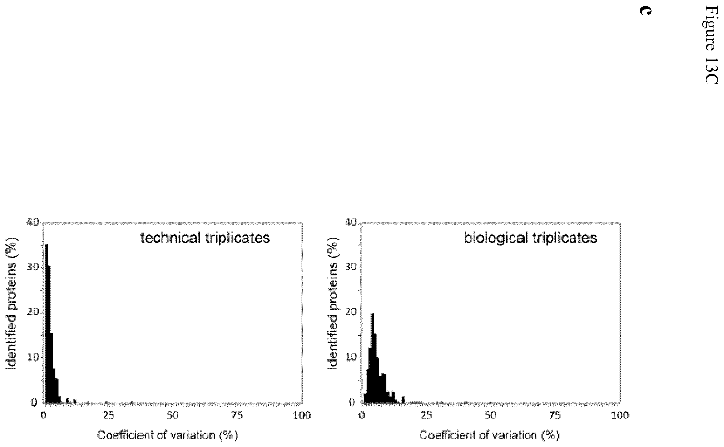

**Figure 13: Validation of SILAC-based protein quantification by measurement of technical and biological triplicates.**
(a) Distribution analysis of $\log_2$ SILAC-protein ratios between non-labelled and SILAC wild type hearts in technical triplicates. The density plots of all three replicates are displayed on the diagonal of the matrix. The diagrams above the diagonal show the $\log_2$-SILAC ratios of the replicates plotted against each other. The Pearson correlation r ranges from 0.93 to 0.96. The slopes, based on median pair wise differences, range from 0.98 to 1.0. The box plots below the diagonal show the distribution characteristics of the direct rations between the replicates. The obtained ratio of ratios gives the relative difference between the light hearts. Whiskers indicate 1.5-fold of the interquartile range (IQR). The IQRs range from 0.09 to 0.1. Compared to the biological replicates (b) higher correlations and smaller IQR are evident reflecting the high accuracy of the SILAC approach. (b) Distribution analysis of $\log_2$ SILAC-protein ratios between non-labelled and SILAC wild type hearts in biological triplicates. The density plots of all three replicates are displayed on the diagonal of the matrix. The diagrams above the diagonal show the $\log_2$-SILAC ratios of the replicates plotted against each other. The Pearson correlation r ranges from 0.61 to 0.85. The slopes, based on median pairwise differences, range from 1.03 to 1.05. The box plots below the diagonal show the distribution characteristics of the direct rations between the replicates. The obtained ratio of ratios gives the relative difference between the light hearts. Whiskers indicate 1.5-fold of the interquartile range (IQR). The IQRs range from 0.18 to 0.22. Compared to the technical replicates (a) lower correlations and larger IQR are evident. (c) Coefficients of variation (CV) were calculated based on relative SILAC label and presented in histograms. Median CV between biological triplicates (4.53%) is about 3-fold higher compared to technical triplicates (1.44%).

## DETAILED DESCRIPTION OF THE EMBODIMENTS

The invention generally relates to a novel method for metabolic labeling of a fish as target organism with a stable isotope.

Before describing in detail exemplary embodiments of the present invention, definitions important for understanding the present invention are given.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of $\pm20$ %, preferably $\pm15$ %, more preferably $\pm10$ %, and even more preferably $\pm5$ %.

It is to be understood that the term "comprising" is not limiting. For the purposes of the present invention the term "consisting of' is considered to be a preferred embodiment of the term "comprising of'. If hereinafter a group is defined to comprise at least a certain number of embodiments, this is meant to also encompass a group, which preferably consists of these embodiments only.

Furthermore, the terms "first", "second", "third" or "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

In case the terms "first", "second", "third" or "(a)", "(b)", "(c)", "(d)" "i", "ii" etc. relate to steps of a method or use or assay there is no time or time interval coherence between the steps, i.e. the steps may be carried out simultaneously or there may be time intervals of seconds, minutes, hours, days, weeks, months or even years between such steps, unless otherwise indicated in the application as set forth herein above or below.

It is to be understood that this invention is not limited to the particular methodology, protocols, reagents etc. described herein, as these may vary. It is also to be understood that the terminology used herein is for the purpose of describing particular embodiments only, and is not intended to limit the scope of the present invention that will be limited only by the appended claims. Unless defined otherwise, all technical and scientific terms used herein have the same meanings as commonly understood by one of ordinary skill in the art.

As has been set out above, the present invention in a first aspect describes a method for metabolic labeling of a target organism with a stable isotope, wherein the stable isotope is transferred from a label source to the target organism

    a) via one or more food chains, each food chain comprising of one or more cascades of ancestor organisms, wherein each cascade forms the ancestor organism for a successor organism of the following cascade, and wherein at least one food chain comprises

        i) a straight food chain comprising at least two cascades, wherein at least two ancestor organisms of the at least two cascades are connected in series; or

        ii) a branched food chain, wherein at least two ancestor organisms of the same or of different cascades are arranged in parallel; or

    b) via one or more one food chains of a) in combination with at least one synthetic diet labeled with the stable isotope as direct label source; or

    wherein the target organism is fish;

wherein the target organism is fed with a diet composition comprising material, cells and/or tissues derived from one or more labeled ancestor organisms of said one or more food chains or a combination thereof; wherein optionally the diet composition further comprises at least one synthetic diet labeled with the stable isotope; and wherein the stable isotope used is $^{13}$C, $^{15}$N, and/or $^{2}$H, or a combination thereof.

The term "isotopic labeling" as used herein refers to a technique for tracking the passage of a sample of substance through a system. The substance is 'labeled' by including or incorporating unusual or stable isotopes in its chemical composition. The isotopes are then detected in a certain part of the system and are derived from the initially labeled substance. In ordinary isotopic labeling two ways of detecting the presence of labeling isotopes are known. Since isotopes have different masses, they can be separated using mass spectrometry. Due to their difference in mass the molecules or substances having incorporated the isotopes have different vibrational modes and can be detected via infrared spectroscopy.

"Metabolic labeling" as used herein is a specific form of isotopic labeling and typically refers to labeling of proteins *in vivo* through metabolic incorporation of a label, for instance, a stable isotope into proteins. It is however conceivable that also other biomolecules such as lipids, carbohydrates, or nucleic acids are labeled. While in chemical (*in vitro*) labeling proteins or peptides are chemically derivatized after protein extraction, metabolic labeling is rather based on the principle that isotope-enriched compounds such as salts, amino acids, or $CO_2$ are provided as a nutrient that can be metabolized by a cell or an organism and incorporated into proteins. It is understood that in metabolic labeling the incorporation occurs at the organism level that is before cell breakage or preparation of the protein mixture. In metabolic labeling experiments samples and controls are typically mixed together in known amounts, whereby one is enriched in stable isotopes. Metabolic labeling can be performed in cells or cell culture as well as in higher organisms such as insects, fish, and mammals. Metabolic labeling thus have the advantage that the stable isotope is introduced into the proteome *in vivo* which allows a "snapshot" of the proteome. Hence, also envisaged by the present invention is to use metabolic labeling for producing a reference organism (control) that is labeled with a stable isotope. The reference or target organism after metabolic labeling contain isotope labeled precursor molecules such as isotope labeled amino acids. The labeled amino acids are metabolically incorporated into the proteins or peptides of the organism. Metabolic labeling generally has the advantage that all peptides of an organism can be used for quantitation in comparative proteomics.

Metabolic labeling comprises methods such as stable isotope labeling with amino acids in cell culture (SILAC) (Ong, S.E., and Mann, M. (2007); Stable isotope labeling by amino acids in cell culture for quantitative proteomics; Methods Mol Biol 359, 37-52.) and stable isotope labeling of amino acids in mammals stable isotope labeling (SILAM) (Wu, C.C. et a al. (2004); Metabolic labeling of mammalian organism with stable isotoptes for quantitative proteomic analysis; Anal Chem 76, 2951-59).

The term "stable isotope labeling with amino acids in cell culture (SILAC)" as used herein describes a common application of stable isotope metabolic labeling, mostly using single cell organisms or mammalian cell in culture, thus limited to cell culture. Typically, the proteins are labeled *in vivo* by introduction of label in the growth medium. SILAC basically relies on metabolic incorporation of a given "light" (unlabeled) or "heavy" (labeled) form of the amino acid into the proteins. When the labeled analog of an amino acid is supplied to cells in culture instead of the natural amino acid, it is incorporated into all newly synthesized proteins. After a certain number of cell divisions, each particular amino acid will be replaced by the isotopic labeled analog. Labeled and unlabeled cells are subsequently mixed, followed by the analysis of the proteome where the intensity of light and heavy variants are compared to obtain relative quantification. The workflow of a SILAC labeling is illustrated in Figure 1A. Typically, a system (B: heavy) is labeled metabolically with one or two heavy amino acids. Quantification of proteins of system (A: light) versus (B: heavy) occurs by mass spectrometry. A subsequent tryptic digestion of the proteomes (A) and (B) reveals pairs of peptides, which differ in the molecular weight of their terminal light and heavy amino acids, e.g. lysines and arginines, respectively.

The terms "stable isotope" as used herein refers to a radioactive or non-radioactive isotopic form of an element, preferably non-radioactive, with identical numbers of protons and electron but having one or more additional neutrons as compared to the standard atom leading to a mass difference. Typically an isotope is considered as being stable, if it has a long half-live. Therefore, also radioactive isotopes with extremely long half-lives may be considered as stable isotopes. Different isotopes of the same element generally have the same chemical characteristics and therefore behave almost identically in biology. One exception is however hydrogen (heavy water). Low concentrations of stable isotopes carrying one or more additional neutrons than standard atoms can be found in all biomolecules. The labeling with stable isotopes is similar to the labeling with radioactive isotopes, but biologically harmless. Therefore they are today an important tool for analytical methods like neutron scattering, NMR-Tomography, NMR- Microscopy, NMR-, Mass-, IR- and Raman-Spectroscopy. These methods are used for the analysis of the structure and dynamics of biomolecules in the fields of biology, pharmacy and medicine, which are important for the development of new drugs. Commonly, stable isotopes used in ecological and biological studies include oxygen, carbon, nitrogen, hydrogen and sulfur. Preferred stable isotopes useful for metabolic labeling as described herein are selected from the group consisting of $^2$H, $^{13}$C, $^{15}$N, $^{17}$O, $^{33}$P, $^{34}$S and combinations thereof. In preferred embodiments of the present invention the labeled isotope use is selected from the group consisting of $^{13}$C, $^{15}$N, $^2$H, and combinations thereof.

The term "target organism" as used herein refers to fishes such as zebrafish or any other economically important fish such as Salmon, amphibians such as *Xenopus* or newt; birds such as chicken; mammals such as a rhodent, a dog, a horse or a human.

In particularly preferred embodiment of the present invention the target organism is a higher organism selected from the group consisting fishes such as *D. rerio* (Zebrafish) or salmon,. In an exemplary embodiment the target organism is *D. rerio* (Zebrafish).

The term "food chain" as used herein refers to a system of one or several cascades of organisms, which can be either organized linearly, i.e. connected in series and herein referred to as "straight food chain", or in parallel, herein referred to as a "branched food chain". It is to be understood that each cascade may comprise at least one or more organisms, at least two or more organisms, at least three or more organisms, at least four or more organisms, at least five or more organisms forming the base for the successor organism in the food chain. The food chain concept as described herein has a certain depth as described by the number of cascades of ancestor organisms and a width of organisms arranged in parallel forming the basis for the following successors of the food chain. Like in the natural food chain habitat which is defined as a linear sequence of links in a food web starting from a trophic organism that eats no other organism in the web and ends at a trophic organism that is eaten by no other organism in the web, a food chain as defined herein comprises a target organism at the top of the food chain, i.e. the organism of the highest cascade and an organism of the lowest cascade which serves as the ancestor organism for a successor organism. In between, there may be one or more ancestor organisms of the same or of different cascades. In other words, each food chain may comprise of at least one or more cascades, at least two or more cascades, at least three or more cascades, at least four or more cascades, at least five or more cascades of ancestor organisms, wherein each cascade of a food chain forms the ancestor organism for a successor organism of the following cascade.

A "cascade" as used herein describes the hierarchy or level where an organism is located within a food chain. In the context of the present invention a stable isotope label can be transferred from a label source to the target organism via one or more cascades of a food chain, *i.e.* upstream. It will be appreciated by one of skill in the art that the organism of the lowest cascade is fuelled with the stable isotope by virtue of a label source. The label source may be synthetic feed, diet, or media comprising the stable isotope. It is to be understood that the organisms of the lowest cascade in turn may serve as feed for a further organism of a higher, subsequent cascade.

Also envisaged by the present invention is that several organisms of the same cascade may be combined in order to transfer the isotopic label directly to the target organism. This concept is herein termed a "branched food chain". Each branch of such a branched food chain may comprise at least one or more cascade, at least two or more cascades, at least three or more cascades, at least four or more cascades, at least five or more cascades. Such a branch of a branched food chain may thus also comprise at least two cascades wherein at least two ancestor organisms of said at least two cascades are connected in series. It is to be understood that the cascades as defined herein may not represent or consider the hierarchical levels of a natural food chain, *i.e.* of the organisms in their natural habitat. For instance, in one specific example of the present invention, labeled mouse tissue may be processed so as to serve as feed for an organism such as a fish or an amphibian, although mouse would not be a member of their natural food chain. Envisaged by the present application is thus a suitable food chain concept, where the label can be effectively transported via several cascades to the target organism.

An "ancestor organism" as used herein refers to a organism which may serve as a feed of the successor organism, *i.e.* may be eaten by an organism of a higher, subsequent cascade. Each cascade except of the highest cascade of a food chain may thus comprise an ancestor organism as defined herein. The ancestor organism of the lowest cascade of each food chain may be fuelled with the isotopic label. "Fuelling" may occur via growing or cultivating an organism of the lowest cascade by synthetic feed or media comprising the isotopic label. It is to be understood that basically any organism that is able to grow on synthetic media or can be fed with a synthetic diet may be an ancestor organism of the lowest cascade as described herein. Envisaged by the present invention are in particular prokaryotic or eukaryotic organisms which can easily be grown on a synthetic medium. In specific embodiments of the present invention the prokaryotic organism is *E. coli.*

In particularly preferred embodiments of the present invention the prokaryotic organism is a cyanobacterium. The term "cyanobacteria" as defined herein also known as blue-green algae, blue-green bacteria, or Cyanophyta refer a phylum of bacteria that obtain their energy through photosynthesis. Suitable cyanobacteria are selected from the classes consisting of *Chroococcales, Gleobacterales, Nostocales, Oscillatoriales, Pleurocapsales,* and *Stigonematales.* A cyanobacterium suitable as an ancestor organism in the context of the present invention may belong to the following genera: *Halospirulina, Planktothicoides, Prochlorococcus, Prochloron, Prochlorothrix, Synecococcus* and *Spirulina.* In further preferred embodiment of the present invention the cyanobacterium is selected from the genus consisting of *Synecococcus* and *Spirulina.*

One envisaged advantage is that cyanobacteria typically contain a better-quality nutrient and vitamin content and are therefore suitable for producing a balanced and biologically competent labeling diet. It is understood that cyanobacteria represent an excellent protein source containing all or almost all essential amino acids. Often cyanobacteria are reliable sources of important vitamins such as Vitamin B12. A further envisaged aspect is that cyanobacteria provide a rich source of long chain polyunsaturated fatty acids (LCPUFA) and a broad range of ions. Furthermore, cyanobacteria can be easily cultivated on synthetic media making them a *bona fide* ancestor organism. A further envisaged advantage is that auxotrophic strains are available making cyanobacteria ideal candidates for amino specific labeling as described herein.

Further suitable non-animal organisms that can be easily cultivated belong to the eukaryotic fungi. Suitable organisms are selected from the phyla Ascomycota or Basidiomycota. In a further preferred embodiment unicellular eukaryote is yeast also termed *Saccharomyces cerevisiae.* Beside the fact that yeast is one of the most popular model organisms for studying genetics and can be also easily cultivated it is suitable in terms of nutritional supplementation. For instance, yeast is known to be an excellent source of protein and vitamins, especially the B-complex vitamins, whose functions are related to metabolism, as well as other minerals and cofactors required for growth. Due to the availability of auxotrophic strains yeast is also a *bona fide* ancestor organism since it can be easily cultivated and labeled.

In a further preferred embodiment of the present invention the ancestor organism of the one or more food chains is prokaryotic or eukaryotic organism such as an animal, a plant or a fungus. Envisaged by the present invention is the metabolic labeling of a target organism using a mammal such as mouse as a labeled ancestor organism. Means and methods for metabolic labeling of rodents have been described in the art (McClatchy, D.B. et al. (2007). 15N metabolic labeling of mammalian tissue with slow protein turnover. J Proteome Res 6, 2005-2010).

In particularly preferred embodiments of the present invention the ancestor organism is mouse, e.g. *Mus musclus* (house mouse). Also envisaged by the present invention is the processing of a mouse that was labeled using suitable synthetic diet in order to obtain a biologically competent feed. Suitable synthetic media for SILAC as well as global uniform labeling are known in the art and are commercially available (e.g. from Silantes GmbH, Munich). Preferably, tissue deriving from parts or organs of the entire organism may be used for producing a labeling feed or diet as defined herein. Also envisaged is the use of specific tissues having a high incorporation rate of the label in order to enhance the labeling efficiency of the feed.

It is also conceivable to use a plant cell as an ancestor organism. It is understood that a plant cell may be easily cultivated on synthetic media. A further envisaged advantage is thus that plant cells may be also of nutritional value and therefore considered for producing a balanced and competent diet. Especially algae can be easily labeled. Suitable algae, without being limited thereto, are selected from the group consisting of *Archaeplastida* such as *Chlorophyta* (green algae), *Rhodophyta* (Red algae), or *Glaucophyta, Rhizaria* or *Excavata* such as *Chloroachniophytes* or Euglenids, *Chromista* or *Alverolata* such as *Cryptophyta,* Dinoflagellates, *Haptophyta,* or Heterokonts like *Bacillariophyceae* (Diatoms), *Bolidomonas, Eustigmatophyceae, Phaeophyceae* (Brown algae), *Chrysophyceae* (Golden algae), *Radphidophyceae, Synurophyceae, Xantophyceae* (Yellow-green algae).

Particular preferred algae are selected from the group of *Chlorophyta,* more preferably *Chlorophyceae* including, but not limited to, *Chetopeltidales, Chetophorales, Chamyomonodadales, Chlorococcales, Chlorocystidales, Microsporales, Oedogoniales, Phaeophilales, Sphaeopleales, Tetrasporales,* or *Volvocales.*

In particularly preferred embodiments of the present invention the plant cell is an algae selected from the group consisting of *Chlamydomonas, Scenedesmus* and *Chlorella.* It is conceivable that algae such as *Chlorella* may be an attractive possible food source because it contains high amounts of protein and other essential nutrients; e.g. about 45% protein, 20% fat, 20% carbohydrate, 5% fiber, and 10% minerals and vitamins. For instance, *Chlorella* is known to be a complete protein source that is further packed with calories, fat, and vitamins thus being suitable as a supplement for obtaining a balanced diet.

"Successor organism" as defined herein refers to an organism of the higher, subsequent cascade located upstream of the ancestor organism, and which may be fed with cells, tissues or material of the ancestor organism. It is also conceivable that the successor organism is fed *ad libidum* with the ancestor organism.

The term "isotope label" as used herein refers to a biomolecule or a substance such as a protein, a lipid, a carbohydrate such as a sugar, or a nucleic acid that has been labeled with a stable isotope as defined herein above. In specific embodiments of the present invention the isotope label is a peptide or a protein. In particular, the amino acids of the peptides and proteins are labeled. Two forms of metabolic labeling of proteins have been described in the art, which are often referred to as "amino acid specific labeling" and "global uniform labeling".

The term "transferred from a label source to said target organism" as used herein means that each labeled ancestor organism may transfer the label indirectly, *i.e.* via subsequent ancestor organism of the higher cascade, and/or directly to the target organism. Envisaged by the present application is a transport of the labeled compound via feeding of the target organism. In other words, the labeled organism transfers its label upstream along the food chain. One envisaged advantage is that the isotope label may be transferred without isotopic dilution. A further important advantage envisaged by the present application is that the organisms forming the basis of a food chain can generally be more easily labeled than the organisms located upstream in the food chain. It is appreciated that the ancestor organism may be administered *ad libidum* similar to their *in vivo* situation.

The term "straight food chain" as used herein refers to a linearly or serially arranged food chain. The concept of a straight food chain is illustrated in Fig. 2A. In a straight food chain the isotope label is thus transferred linearly (or serially) via at least two ancestor organisms (Ancestor 1c and 2c) of at least two consecutive cascades (Cascade 1 and 2) to the target organism. In a straight food chain the isotope label may be transferred linearly via at least two ancestor organisms, at least three ancestor organisms, at least four ancestor organisms, at least five ancestor organisms of at least two consecutive cascades, at least three consecutive cascades, at least four consecutive cascades, at least five consecutive cascades to the target organism. Without being bound to a theory, accumulation of the isotope label is facilitated with increasing cascades, so that the ancestor organism which may directly serve as a feed for the target organism already has a high incorporation rate of the stable isotope. It is thus conceivable that feeding of the target organism with a higher order ancestor organism of a straight food chain as described herein facilitates an effective and quick incorporation, so that less time may be required in order to obtain a labeled organism with a complete incorporation rate.

The term "branched food chain" as used herein may comprise at least two or more labeled ancestor organisms, at least three or more labeled ancestor organisms, at least four or more labeled ancestor organism, at least five or more labeled ancestor organisms, which are arranged in parallel. The two parallel ancestor organisms may be located in the same or in different cascades. A branched food chain is depicted on the left panel in Fig. 2A. A branched food chain thus requires that at least two ancestor organisms (Ancestor 1a and 1b) that are not linearly and serially arranged and e.g. are arranged next to each other. For instance, two labeled organisms of the lowest cascade may represent a branched food chain and may directly serve as a feed for the target organism. The underlying rational of a branched food chain is to broaden the spectrum of available food resources. Without being bound to a theory a branched food chain opens up the possibility to combine different supplies of food so as to generate a balanced and biologically competent feed for labeling of the target organism. Also envisaged by the present invention is thus the provision of an economic collection of organisms of the food chain in order to obtain a balanced diet.

A "balanced diet" means that the feed for metabolic labeling as described herein does not essentially differ from established feeds with respect to growth of the targeted organism and its breeding capabilities. Envisaged is thus a feed composition that resembles or reflects the physiological situation of the targeted organism in its natural habitat. An example for a balanced diet feed composition according to the present invention is depicted in Fig. 8 and described in Example 1. The exemplary SILAC diet for zebrafish contain Lys-6 labeled cells from a lysine auxotroph *E. coli* strain, a lysine auxotroph *S. cerevisiae* strain, SILAC mouse tissue, and a SILAC mouse diet. The diet comprises a mixture of different ancestor organisms of the same cascade and thus form a branched food chain as described herein. It could be shown that the SILAC diet has no physiological consequences on adult zebrafish. During an observation period of 5 weeks, all fishes showed no loss of weight and no obvious changes in the locomotor activity compared to the regular diet. It could thus be concluded that the composition of the diet and the Lys-6 labeling is appropriate for labeling of zebrafish.

Also envisaged by the present invention is a balanced diet composition that is tailored to the developmental stage of the target organism thereby taking into account the nutritional requirements of each stage. It is conceivable that a complete labeling may require a feeding over several generations so that it could be demanding to provide a suitable balanced diet also for a target organism in its initial stages, e.g. embryonic or larval stages. As shown in the right panel in Fig. 8B the diet for larval zebrafish essentially differs in its composition from adult zebrafish diet.

The exemplary diet composition also demonstrates that a synthetic diet that is non-specific for zebrafish, namely a mouse synthetic diet, may also be advantageously used as a further label source in addition to a food chain combination. The synthetic diet may complement essential nutrients required for growth of the target organism, if the nutritional supply provided by the organisms of the food chain are not sufficient. An exemplary embodiment is thus to use the synthetic diet for fine tuning of the nutritional source in order to obtain a balanced diet as defined herein.

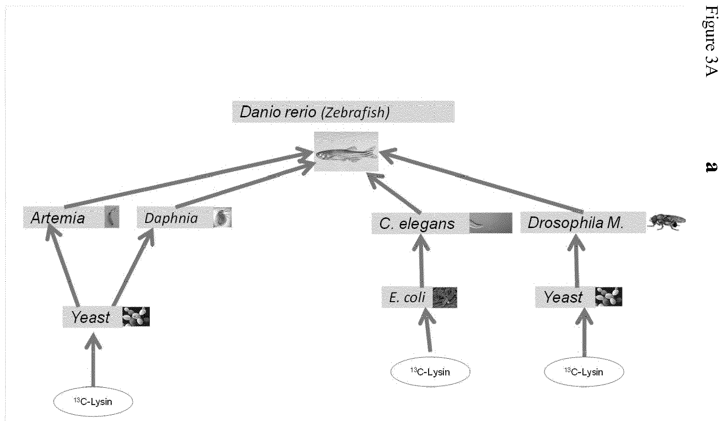

Also envisaged by the present application are combinations of straight and branched food chains which may be combined in form of modules as can be seen in Fig. 2B. According to the method described herein, at least one food chain may be a branched food chain or a straight food chain as indicated by the circles. Exemplary feed compositions for the labeling of a target organism of interest such as zebrafish (*D. rerio*) and an amphibian based on the combination of different food chain modules are illustrated in Fig. 3A and 3B. As can be seen in Fig. 3A, the ancestors of the lowest cascade (Cascade 1) are fuelled by $^{13}$C-lysine as label using lysine-auxotrophic strains of yeast (*S. cerevisiae*) and *E. coli,* respectively. These primary ancestor organisms (yeast, *E. coli*) may subsequently serve as a feed for the organisms of the following cascades (successor organisms). The ancestor organisms of the second cascade (*Artemia, Daphnia, C. elegans, Drosophila*) themselves represent the direct ancestor and may serve as a feed for the target organism. It is to be understood that a combination of several straight food chains as depicted in Fig. 3A also represents a branched food chain that may be used for producing a biologically competent feed for the target organism as described herein.

A more complex example of a food chain is shown in Fig. 3B where the target organism is an amphibian. In the natural food chain habitat, amphibians are typically located one cascade higher than zebrafish. The exemplary feed compositions are based on a further feed source using tissue from mouse, which was labeled with synthetic $^{13}$C-lysine medium. In this Example, labeled Zebrafish itself may be an ancestor for the amphibian. Also envisaged by the present invention is the use of each consecutive ancestor organism of a straight food as defined herein for producing a feed for the target organism. For instance, yeast, Daphnia, and Zebrafish may be combined to yield a balanced feed for metabolic labeling of an amphibian.

In preferred embodiments of the present invention a labeled higher eukaryote such as mouse may be used as an ancestor organism for feeding a target organism, which would not be a related to its natural food chain. It is envisaged by the present invention that tissues of basically any higher eukaryotic organism may be used as feed for the target organism.

In particularly preferred embodiments of the present the target organism is fed with a diet composition comprising material, cells and/or tissues derived from one or more labeled ancestor organisms of one or more food chains and/or cells from one or more cell cultures, or a combination thereof.

The present invention also describes the provision of different sources of food labels. As depicted in Fig. 4A the organisms of the lowest cascade may be fuelled by media comprising differently labeled compounds. In the Example according to Fig. 4A algae such as *R. eutropha* and *Spirulina* may be grown on $^{13}$CO$_2$ and/or $^{15}$N-salts. Means and methods for isotopically label an organism with $^{13}$CO$_2$ are known to the skilled person (WO 2000/012140; Method for labeling biopolymers using isotopes).

In specific embodiments of the present invention the ancestor organisms may be also a non-animal such as a prokaryote, *e.g. E. coli* or a cyanobacterium, or a eukaryotes such as fungi, e.g. yeast, a plant algae, or a plant. As mentioned above such a combination provides a broader spectrum of supply of food resulting in a balanced feed with differently labeled compounds for metabolic labeling of a single organism. The skilled person knows means and methods to measure the requirement of a competent food, *i.e.* to determine the compounds that are essential for proper growth of the target organism and may thus combine the food chains accordingly in order to obtain a balanced feed.

The feed composition used as a feeding source for labeling of the target organism may not be restricted to one or more food chains as described herein. A combination of one or more food chains with a synthetic diet are is also envisaged by the present invention.

"Synthetic diet", "synthetic feed" or "synthetic media" may be used interchangeably and refer to a labeled feed suitable for labeling of an organism by feeding or administration, in which either a specific amino acid is fully or at least to a large extent replaced by their heavy isotopic counterpart.

The term "large extent" in this context means that at least 20%, 25%, 30%, 35%, or 40%, more preferably 45%, 50%, 55%, 60%, 65%, or 70%, even more preferably at least 75%, 80%, 85%, or 90%, most preferably at least 95, 96%, 97%, 98%, or 99%, or any value in between of a specific amino acid or a specific atom are replaced by the heavy isotope.

Typically, the synthetic diet used for metabolic may comprise either labeled (heavy diet) or unlabeled compounds (light diet). The differential labeling using heavy and light diets leads to either a "heavy" and a "light" labeled organism, respectively which may be used for comparative proteomics analysis.

In particularly preferred embodiments of the present invention the metabolic labeling is an amino acid specific labeling. The term "amino acid specific labeling" as used herein refers to a labeling method where only specific amino acids, commonly one, two three, four, five, six or more amino acids are tagged using a stable isotope. In principle, any of the 20 naturally occurring amino acids could be used as a precursor for labeling proteins. In particularly preferred embodiment of the present invention one or more amino acids are labeled.

Especially in animal metabolism, a subset of the amino acids can be synthesized by the organism themselves (non-essential amino acids). Those that cannot be synthesized must be provided in form of a diet (essential amino acids). Typical essential amino acids are histidine, isoleucine, leucine, lysine, methionine, phenylalanine, threonine, tryptophane, and valine. If the amino acid is non-essential, then the cell or organism is capable of synthesizing that amino acid from precursors that are not themselves labeled, which has the effect of reducing the relative isotope abundance of the precursor pool (isotopic dilution). It is thus conceivable to use essential amino acids if an isotopic dilution of the label shall be avoided or minimized. Also envisaged by the present invention is also the use of non-essential amino acids. It will be appreciated by the skilled artisan that some amino acids such as arginine are non-essential but nevertheless obligatory for cells and are often provided in the cell culture medium because a depleted arginine pool by protein synthesis could compromise essential metabolic cycles in the cell.

In particularly preferred embodiments of the present invention the labeled amino acid is lysine and/or arginine. In particularly preferred embodiments of the present invention $^{13}$C-labeled lysine is used for amino specific labeling.

Also envisaged by the present invention is the provision of more than one label in a target organism. In specific embodiments of the present invention at least two, at least three, at least four, at least or at least six amino acids are specifically labeled in the target organisms. In such a multiply labeled organism each label may be used for assessment of a specific state or condition of the organism. The provision of such multiply labeled organisms mainly has the advantage that several states or conditions of a cell or organism may be investigated simultaneously in one single analysis, that means without the need for carrying out several approaches or generating several labeled organisms which saves costs and time.

In particularly preferred embodiments of the present invention the metabolic labeling is global uniform labeling. The term "global uniform labeling" as used herein refers to an unspecific labeling method where all amino acids of an organism are labeled. Commonly, $^{13}$C or $^{15}$N are used for the complete labeling of a biological system. It is however appreciated that also $^{2}$H may be used for global uniform labeling. In particularly preferred embodiment of the present invention the labeled isotope used is $^{13}$C, $^{15}$N, and/or $^{2}$H, or a combination thereof.

Typically, global uniform labeling of prokaryotic and eukaryotic cells is based on synthetic media in which the compounds containing N- and C-atoms are replaced by the corresponding heavy isotope-labeled compound. Such compounds may be e.g. labeled salts, carbohydrates, or amino acids.

Global $^{15}$N-*in vivo* labeling of mice has been described in the art (Wu and MacCoss (2007). Quantitative proteomic analysis of mammalian organisms using metabolically labeled tissues. Methods Mol Biol. 359, 191-201). In particular, mice were labeled using media in which the protein component is replaced by a $^{15}$N-labeled protein hydrolysate that was obtained from $^{15}$N-labeled cyanobacteria or algae. Typically, the isotope label, e.g. $^{15}$N, is incorporated into the $\alpha$-amino group. Proteomics studies on rats labeled uniformly with $^{15}$N have been previously reported (D. B. McClatchy et al. (2007); 15N metabolic labeling of mammalian tissue with slow protein turnover, Journal of Proteome Research, 6(5), 2005-2010) using $^{15}$N-labelled algae as a protein source.

In specific embodiments of the present invention a labeled mixture of amino acids or a labeled protein hydrolysate is used as a protein source for global uniform labeling. In a further preferred embodiment of the present invention the protein source is a labeled protein hydrolysate.

The term "enrichment of the stable isotope" as used herein refers to the label efficiency, *i.e.* the rate of incorporation of the stable isotope into the organisms to be labeled. Typically, a high enrichment is required for proper quantitation in SILAC and SILAM as defined herein. In SILAC experiments the average labeling efficiency the incorporation efficiency can be generally calculated on the peptide level as:

$$1 - 1 / \text{Ratio (H / L)},$$

wherein H is defined as the amount of "heavy peptide" and **L** is defined as the amount of "light" peptide.

The incorporation rate is calculated as

$$\% \text{ label} = (\text{SILAC-ratio}*100) / (\text{Silac-ratio}+1).$$

For example, a SILAC-ratio of 10 would lead to a 91% incorporation rate.

Envisaged by the present invention is a target organism that is completely or almost completely labeled. In particularly preferred embodiments of the present invention the enrichment of the stable isotope in the target organism is at least about 70%, 75%, 80%, or 85%, even more preferably at least about 90%, 91%, 92%, 93%, 94%, 95%, or 96%, and most preferably at least about 97%, 98%, 99% or 100%. Using the means and methods as described herein, an almost complete labeling was achieved during the growth of the F1 and all subsequent generations (see Example 1). "Completely labeled or almost completely labeled" means that the target organism enrichment of the stable isotope in the target organism is at least about 90%, preferably 91%, 92%, or 93%, more preferably 94%, 95%, or 96%, and most preferably at least about 97%, 98%, 99% or 100%. In particularly preferred embodiments a completely or almost completely labeled organism may be used as a spike-in standard in quantitative proteomics. If the target organism is used for pulse SILAC labeling as described hereinafter a much lower labeling efficiency is sufficient. In such a case the labeling efficiency may be at least about 30%, 40%, or 50%, preferably at least about 55%, 60%, or 65%, more preferably at least about 70%, 75%, 80%, or 85%, even more preferably at least about 90%, 91%, 92%, 93%, 94%, 95%, or 96%, and most preferably at least about 97%, 98%, 99% or 100%.

In particular preferred embodiments, it may be also sufficient or useful if only parts of the organism is fully labeled. As shown in Fig. 9, the SILAC ratios of the various organs tested may vary. Referring to Example 1 and Fig. 9, the fastest Lys-6 incorporation was measured for liver and gill tissue due to higher metabolic rates and cell proliferation as compared to other tissues.

In a preferred embodiment of the present invention the direct ancestor organism of the at least one food chain used for transferring the label to said target organism has an average SILAC label of at least 20%, 30%, 40%, or 50%, preferably of at least 60%, 65%, 70%, or 75%, more preferably of at least 80%, 85%, or 90%, even more preferably of at least 91%, 92%, and most preferably of at least 97%, 98%, 99% or 100%. It will be appreciated by the skilled person to use only parts of an organism that has a high incorporation rate in order to increase the labeling efficiency of the feed. It may be thus be useful to process only specific tissues which complete or almost complete SILAC ratio.

The skilled person is also aware of means and methods for determining the labeling efficiency in global uniform labeling as defined herein. Typically, labeling efficiency in global uniform labeling is determined via the comparison of the theoretical mass of a peptide or a protein and the actual measured mass.

In another preferred embodiment of the present invention the direct ancestor organism of said at least one food chain used for transferring the label to said target organism has an average global uniform label of at least 30%, 40%, or 50% preferably of at least 55%, 60%, or 65%, more preferably of at least 70%, 75%, 80%, or 85%, even more preferably of at least 90%, 91%, 92%, 93%, 94%, 95%, or 96%, and most preferably of at least 97%, 98%, 99% or 100%, or any value in between.

The present invention also describes the use of one or more cell cultures labeled with a stable isotope instead of or in addition to the one or more food chains as defined herein for metabolic labeling of a target organism. It is to be understood that a cell culture is an equivalent to an ancestor organism, i.e. may also serve as a suitable food source for feeding of a successor organism or target organisms as defined herein above. It will be appreciated by the person skilled in the art that the isotope label may be effectively transferred to the target organism via a cell culture. Also envisaged by the present invention is to use one or more cell cultures in combination with synthetic diet as defined herein above for the metabolic labeling of higher eukaryote.

While the prior art exploited labeled cell cultures directly for quantitative proteomic approaches, the use of labeled cell cultures as a competent feed in the sense of the present application has not been described. It will be appreciated by one of skill in the art that labeled cell cultures may be advantageously used for fuelling a food chain as described herein and may thus help to effectively transfer the isotope label to the target organism.

The one or more cell cultures may be selected from the group consisting of a plant cell culture, an insect cell culture or a mammalian cell culture. The one or more cell cultures may be a combination of one or more, two or more, three or more, four or more, five or more mammalian cell cultures and/or one or more, two or more, three or more, four or more, five or more plant cell cultures and/or one or more, two or more, three or more, four or more, five or more insect cell cultures.

The mammalian cell culture may be a HeLa cell culture.

Also envisaged by the present application is the direct administration of the labeled cell cultures. One envisaged advantage is that cell cultures can be easily grown on suitable media and further provide a certain nutritional value for the successor organism as defined herein. Also envisaged is thus a combination of cell cultures with one or more food chains for metabolic labeling of a target organism.

The skilled person is aware of the various means and methods for stable isotope metabolic labeling of cell cultures. The principle of the SILAC labeling as described have been described in the art (Ong, S.E., and Mann, M. (2007). Stable isotope labeling by amino acids in cell culture for quantitative proteomics. Methods Mol Biol 359, 37-52) and is illustrated in Figure 1A.

In a second aspect, the present invention discloses a method of producing a diet for metabolic labeling of a target organism with a stable isotope, comprising

i) producing one or more ancestor organisms of a straight food chain comprising at least two cascades, wherein the one or more ancestor organisms are labeled with a stable isotope, and wherein at least two labeled ancestor organisms are connected in series, and wherein at least one labeled ancestor organism is an organism of at least the second or higher cascade; and/or

ii) producing at least two ancestor organisms of a branched food chain, wherein the at least two ancestor organisms are each labeled with a stable isotope and wherein the at least two labeled ancestor organisms of the same or of different cascades are arranged in parallel; and

iii) processing said one or more labeled ancestor organisms obtained in i) and/or ii) and/or said one or more labeled cell cultures obtained in iii) to obtain a diet for feeding the target organism;

wherein the target organism is fish;
wherein the target organism is fed with a diet composition comprising material, cells and/or tissues derived from one or more labeled ancestor organisms of said one or more food chains or a combination thereof; wherein optionally the diet composition further comprises at least one synthetic diet labeled with the stable isotope; and wherein the stable isotope used is $^{13}C$, $^{15}N$, and/or $^{2}H$, or a combination thereof.

It is to be understood that the definitions and preferred embodiments as set out for first aspect of the invention, namely a method for metabolic labeling of a target organism, also apply to the second and further aspects of the present invention.

As has been set out in detail above, the basic principle underlying the present invention is the use of food chains as described herein above, capable of effectively transporting the label of interest to a target organism. The present invention describes means and methods how this transport can effectively be achieved, namely by virtue of producing a suitable diet or feed. Envisaged by the present invention is also the generation and processing of a suitable ancestor organism thereby taking into account the SI food chain concept as described herein.

It will be appreciated by the skilled person that several or all ancestor organisms may contribute in the production of a balanced and biologically competent feed as defined herein above. In further preferred embodiments of the present invention the balanced diet comprises a combination of two or more ancestor organisms of different cascades. It is to be understood that the food chain may at least fulfill the minimal requirements as described herein (see circles Fig. 2A and 2B). For instance, if only one ancestor organism is used, it is required that the ancestor organism is an organism of "at least the second or higher cascade". It is to be understood that there are various possibilities to produce the ancestor organism of the at least the second or higher cascade which will depend on the feasibility producing the ancestors. In particularly preferred embodiments of the present invention the ancestor organism of the lowest cascade is fuelled with isotope label using a synthetic medium.

The term "processing said one or more labeled ancestor organism" as used herein refers to a mixing, shredding, chopping, lyophilization of the material deriving from an ancestor organism. The skilled artisan is aware of the numerous methods for processing an organism or parts thereof so as to result in a suitable feed. Within the context of the present invention the material may be the whole organism or parts thereof such as specific organs, tissues, or cells. Also envisaged by the present invention is the administration of the ancestor organism *ad libidum, i.e.* a direct feeding of the organism without processing. The envisaged advantage is that this form of feeding largely resembles the situation *in vivo*.

An example for such a processing is described in Example 1, where tissues of a labeled mouse including parts from the skeletal muscle liver, as well as white adipose tissue was chopped, lyophilized and mixed with fully labeled *E. coli, S. cerevisiae* and SILAC mouse diet. In this specific example, the mixing ratio of the dry weight was 3 (mouse tissue): 2 (mouse diet): 1 (*E.coli*): 1 (yeast). It is however conceivable that the mixing ratio may vary and primarily depends on the overall nutritional status of each component used for the feed composition. As set out above, the feed composition may also depend on the developmental stage of the organism to be labeled.

In yet another preferred embodiment of the present invention the balanced diet comprises cells and/or tissues derived from one or more labeled ancestor organisms of one or more food chains and/or cells from one or more cell cultures. In particularly preferred embodiments of the present invention the method of producing a diet for metabolic labeling comprises adding a synthetic diet comprising the stable isotope label as an additional label source.

In one specific exemplary embodiment of the present invention larval and young zebrafish were fed using a specific heavy diet with smaller particle size and higher content of the bacteria and yeast fraction (see Fig. 8). The size of the processed material, e.g. particle size may thus be considered, when producing a biologically competent feed. The particle size may depend on the target organism as well as on the developmental stage (Fig. 8C and D).

It will be appreciated from the above that the method of producing a diet depends on the form of metabolic labeling. In a preferred embodiment of the present invention the metabolic labeling is an amino acid specific labeling. In yet another preferred embodiment of the present invention the ancestor organism of the lowest cascade is an auxotroph.

In further specific embodiment the metabolic labeling is a global uniform labeling by a labeled amino acid mixture or a labeled protein hydrolysate.

In a further preferred embodiment of the present invention the target organism is *D. rerio* (Zebrafish) or an economically important fish. Of particular interest is zebrafish since it is functionally similar to humans and represent a bona fide model for studying development, disease and tissue regeneration. Also envisaged by the present invention are economically important fish such as salmon. One potential field is the improvement of productivity. Also envisaged is the provision of biologically competent feed for the metabolic labeling of dogs. Dog is of particular interest since it is a model organism for behaviorism. Possible application may also be in the field of veterinarian or human medicine for diagnostic purposes.

In a further aspect, the present invention describes a diet composition for metabolic labeling of a target organism obtainable by the method according to a second aspect of the present invention.

In yet a further aspect, the present invention relates to a diet composition for metabolic labeling of a target organism with a stable isotope, wherein the diet composition comprises material, cells, and/or tissues of

> i) at least one ancestor organism of at least the second cascade of a straight food comprising at least two cascades, wherein the at least one ancestor organism is labeled with a stable isotope and wherein at least two labeled ancestor organisms are connected in series;
> ii) at least two ancestor organisms of a branched food chain, wherein the at least two ancestor organisms are each labeled with a stable isotope and wherein said at least two labeled ancestor organisms of the same or of different cascades are arranged in parallel; and/or

wherein the target organism is fish;
wherein the target organism is fed with a diet composition comprising material, cells and/or tissues derived from one or more labeled ancestor organisms of said one or more food chains or a combination thereof; wherein optionally the diet composition further comprises at least one synthetic diet labeled with the stable isotope; and wherein the stable isotope used is $^{13}$C, $^{15}$N, and/or $^2$H, or a combination thereof.

wherein the target organism is fish;
wherein the target organism is fed with a diet composition comprising material, cells and/or tissues derived from one or more labeled ancestor organisms of said one or more food chains or a combination thereof; wherein optionally the diet composition further comprises at least one synthetic diet labeled with the stable isotope; and wherein the stable isotope used is $^{13}$C, $^{15}$N, and/or $^2$H, or a combination thereof.

In a preferred embodiment of this aspect the diet composition further comprises at least one synthetic diet labeled with the stable isotope.

In specific embodiments of the present invention, the diet composition is suitable for metabolic labeling of zebrafish. In further specific embodiment of the present invention the diet composition for metabolic labeling of zebrafish comprises Lys-6 labeled *Escherichia coli, Saccharomyces cerevisiae,* SILAC mouse tissue as described herein above and a SILAC mouse diet (Silantes GmbH, München).

Another aspect of the invention is the use of the diet composition according to the present invention for producing a metabolically labeled target organism as a reference for a quantitative proteomic approach.

Another aspect of the invention is the use of a target organism labeled by the method of metabolic labeling according to the present invention as a spike-in standard in quantitative proteomics approaches. The term "spike-in standard" as used herein refers to a reference that may be present in form of a organism or parts thereof such as organs, tissues, or cells that is labeled with one or more stable isotopes as described herein above. "Spike-in" means that the labeled organism or parts thereof is admixed to a non-labeled organism or parts thereof. The skilled person is aware of how to make use of spike-in standards in quantitative proteomics. Such a "spike-in" standard is typically used for the direct comparison of different states or conditions or as a scale factor for the comparison of two different states.

In one envisaged example, two organisms A and B are differentially labeled. One organism (A) has incorporated one or more isotopic labels, *i.e.* the labeled target organism, while the second organism (B) remains unlabeled. The differential labeling can be achieved by using a heavy and light feed based on the food chain concept as described herein above. It will be immediately appreciated by the skilled person that the differentially labeled organisms may correspond to different states or conditions. For instance, one organism may suffer from a certain disease, while the other organism may be the healthy counterpart. The differentially labeled organisms are admixed and subjected to quantitative analysis.

Also envisaged are approaches, where two non-labeled organisms A and B or parts thereof are assigned to different states or conditions and the labeled target organism or parts thereof is provided as the spike-in standard C. In such a case, the labeled organism C is spiked-in to each of the organisms A and B and the mixture subjected to quantitative analysis. The organism C thus serves as a scale or reference factor for calculating the amounts of peptides or proteins being present in the different states A and B.

Another aspect of the invention is the use of a target organism labeled by the method of metabolic labeling according to the present invention for pulse SILAC labeling. The term "pulse SILAC labeling" as used herein refers to the labeling of a target organism over a certain time period. This approach is suitable for studying the time dependent proteome pattern. Typically, such a pulse SILAC labeling may be used to obtain information on the protein turnover in an organism. Without being limited thereto, one envisaged example is provided herein in Example 6, where the incorporation of Lys-6 into regenerating fins was analyzed over time.

Hence, pulse SILAC labeling can be used to identify the rate of newly synthesized proteins, e.g. during regenerative processes. It will be immediately appreciated by the person skilled in the art that such an approach may allow for the detection of novel cell regulating factors such as transcripton factors, deacetylases, and kinases. Typically, pulse SILAC labeling can be achieved by switching of the biological competent diet used for labeling. For instance, it is conceivable to start with a light diet comprising a light isotope and switch to a diet comprising the heavy isotope. Subsequently, the incorporation rate of the heavy isotope is measured over time.

A further envisaged example is the measurement of isotope dilution over time. In such an approach, a target organism already having incorporated one or several heavy isotopes is fed with a light diet and the loss of incorporated heavy isotope is measured over time (isotope dilution).

As has been demonstrated herein pulse SILAC labeling may be carried for each specific tissue or organ in order to obtain global proteome -wide information on protein-turnover.

## EXAMPLES

## Example 1: Labeling of zebrafish with $^{13}C_6$-Lysine:

For the experiments the local zebrafish strain "Bad Nauheim (BNA)" and the transgenic line *Tg(cmlc2:egfp)* (Huang, C.J., et al. (2003). Germ-line transmission of a myocardium-specific GFP transgene reveals critical regulatory elements in the cardiac myosin light chain 2 promoter of zebrafish. Dev Dyn 228, 30-40) were used. Adult and embryonic zebrafish were maintained under standard laboratory conditions at 28°.

To label zebrafish a heavy diet based on Lys-6 labeled *Escherichia coli, Saccharomyces cerevisiae* (Silantes GmbH, Munich), SILAC mouse tissue and SILAC mouse diet (Silantes GmbH, Munich) was developed and used.

The lysine auxotroph *E. coli* DSM1099 was pre-cultured twice over night in a small volume of M9 media (Sigma-Aldrich) which was supplemented with 2 g/l glucose (AppliChem), 0.49 g/l $MgSO_4 \cdot 7H_2O$ (AppliChem), 0.015 g/l $CaCl_2 \cdot 2H_2O$ (AppliChem), 2.53 g/l drop-out amino acids without lysine (Formedium) and 0.05 g/1 $^{13}C_6$-Lysine (Silantes). For large-scale culturing, the second pre-culture was diluted 1:100 in supplemented M9 media and incubated at 30°C until reaching the stationary phase. Finally, Lys-6 labeled *E. coli* were harvested and freeze-dried by a lyophilizer (Christ).

Labeled yeast was obtained from Silantes. As an additional component we used mouse tissue, including parts from the skeletal muscle liver, as well as white adipose tissue. Mouse tissue was chopped, lyophilized and mixed with fully labeled *E.coli, S. cerevisiae* and SILAC mouse diet. The mixing ratio of the dry weight was 3 (mouse tissue): 2 (mouse diet): 1 (*E.coli*): 1 (yeast). To feed larval and young zebrafish, we used a heavy diet with smaller particle size and higher content of the bacteria and yeast fraction (**Figure 8**). All zebrafish were fed semi-daily with the heavy diet and during the first 6 weeks we supplemented regular non-labeled fish food (SDS100-200, Special Diets Services).

To generate the SILAC zebrafish a diet containing Lys-6 labeled cells from a lysine auxotroph *Escherichia coli* strain (DSM1099), a lysine auxotroph Saccharomyces cerevisiae strain, SILAC mouse tissue, and SILAC mouse diet was developed (**Figure 8**) In the study only $^{13}C_6$ lysine (Lys-6) labeled cells were used because arginine is in organisms like yeast and fly converted into other amino acids and most likely also in fish (Bicho, C.C. et al. A genetic engineering solution to the "arginine conversion problem" in stable isotope labeling by amino acids in cell culture (SILAC). Mol Cell Proteomics 9, 1567-1577; Sury, M.D., et al. The SILAC fly allows for accurate protein quantification in vivo. Mol Cell Proteomics 9, 2173-2183). Therefore, the endoproteinase LysC was used for all subsequent protein digestions to get quantifiable peptides with a C-terminal lysine residue. First, it was tested whether the SILAC diet has any physiological consequences on adult zebrafish. During an observation period of 5 weeks, all fishes showed no loss of weight and no obvious changes in the locomotor activity compared to the regular diet. It was thus concluded that the composition of the diet and the Lys-6 labeling is appropriate to label zebrafish. To monitor the proper Lys-6 incorporation over time several tissues were isolated each week for 5 weeks. After protein isolation and in solution digestion with LysC, samples were analyzed by liquid chromatography mass spectrometry.

The incorporation of Lys-6 of several tissues was measured (**Figure 9**) and for the unsaturated incorporation rates measured SILAC ratios were calculated as % SILAC ratio [% label = (SILAC-ratio* 100)/ (Silac-ratio+1)]. For example, a SILAC-ratio of 10 would lead to a 91% incorporation rate. We observed the fastest Lys-6 incorporation for liver and gill tissue indicating higher metabolic rates and probably more cell proliferation compared to other tissues.

In contrast, tissues with lower turnover such as the swim bladder and eyes showed a weaker Lys-6 incorporation (**Fig. 5a**). For example, one of the most abundant lens proteins the crystallines showed almost no Lys-6 incorporation (**Fig. 10**).

In contrast, proteins which were localized in the retina, like Opsin and Rod transducin showed much higher Lys-6 incorporation (∼40%) during the feeding period. This shows that the incorporation is not only dependent on the intrinsic protein stability ($T_{1/2}$) but also on the tissue turnover and organ substructures.

Along that way, proteins isolated from the blood showed also a high variation of their Lys-6 incorporation. For example, secreted serum proteins like the Vitellogenin, Apolipoprotein, and Transferrin were detected with a SILAC labeling efficiency of up to 70% after the 5 weeks feeding period, whereas proteins from erythrocytes like Haemoglobin and the Carbonic anhydrase I were labeled to a much lower extend, indicating the long half live for erythrocytes.

Taken together, SILAC incorporation can be used to profile global tissue turnover and it helps to determine the origin of proteins from different cellular pools.

## Example 2: Dissection of adult zebrafish organs and preparation of embryonic zebrafish hearts (experimental setup)

To dissect organs of adult animals, zebrafish were anaesthetized with 0.1% Ethyl-3-aminobenzoate-methanesulfonic acid salt (Tricaine, Sigma-Aldrich) solved in water. Skin and body wall muscle were carefully removed to expose internal organs like heart, liver or swim bladder. Organs were removed, shortly washed in ice-cold PBS and frozen in liquid nitrogen. Homozygous embryos of the transgenic line *Tg(cmlc2:egfp)* were anaesthetized at 72 hpf and 120 hpf. To isolate heart tissue, embryos were pipetted through a 1.1 mm-needle. Separated GFP-positive ventricles were identified under fluorescent light, collected, and frozen in liquid nitrogen.

## Example 3: Immunohistochemistry

Embryos at 72 hpf were gently washed in PT (0.3% Triton X100 in PBS pH 7.3) and fixed in 4% paraformaldehyde (PFA)/PBS over night. Whole-mount staining was performed in PBT (4% BSA, 0.3% Triton X-100, 0.02% NaN$_3$ in PBS pH 7.3) using mouse monoclonal antibody zn-8 (Hybridoma Bank) in a dilution of 1:10 and AlexaFluor-conjugated secondary antibody (Invitrogen) which was diluted 1:200.

## Example 4: Sample preparation and Mass Spectrometry

Dissected organs were homogenized in SDS lysis buffer (4% SDS in 100mM Tris/HCl pH 7.6) using an Ultra-Turrax (Ika) or a glas douncer (Kontes glass company). For complete lysis, samples were shortly heated at 95°C. After DNA shearing by sonication, the lysates were clearified by centrifugation at 16,000 g for 5 min. Protein concentration was estimated using the DC protein assay (Biorad). To check incorporation of Lys-6 in different zebrafish organs, samples were digested in solution as described in previously (Ong and Mann (2006); identifying and quantifying sites of protein methylation by heavy methyl SILAC. Cur Protc Protein Sci. 2006 Dec Chapter 14: Unit 14.9.)

In brief, proteins were precipitated using 4 volumes of ice-cold acetone and the protein pellet was dissolved in 6 M urea/2 M thiourea, 10 mM HEPES pH 8. Next, proteins were reduced with 1 mM dithiothreitol (DTT), alkylated with 5 mM iodoacetamide and digested with the endopeptidase Lys-C (Wako). Peptides were purified by stop and go extraction (STAGE) tips (Rappsilber, J., Ishihama, Y., and Mann, M. (2003) Stop and go extraction tips for matrix-assisted laser desorption/ionization, nanoelectrospray, and LC/MS sample pretreatment in proteomics. Anal Chem 75, 663-670). For large-scale studies, lysates were loaded on a SDS-PAGE (NuPAGE 4%-12% Bis-Tris gel, Invitrogen) and separated proteins were stained with Colloidal Blue Staining Kit (Invitrogen). In-gel digestion of evenly sized gel pieces was performed as described in previously (Shevchenko, A., et al. (2006). In-gel digestion for mass spectrometric characterization of proteins and proteomes. Nature protocols 1, 2856-2860). Alternatively, samples were subjected to the filter aided sample preparation (FASP) protocol combined with OFFgel fractionation (Wisniewski, J et al. (2009). Combination of FASP and StageTip-based fractionation allows in-depth analysis of the hippocampal membrane proteome. J Proteome Res 8, 5674-5678). In brief, proteins were loaded on a filter unite for exchanging buffer, reduction/alkylation and digestion with LysC. After desalting, peptides were separated by isoelectric focusing using an OFFgel fractionator (Agilent) according to the manufactures guidelines.

Reverse-phase nano liquid chromatography (LC) was performed by using an Agilent 1200 nanoflow or Proxeon LC system. The LC system was coupled to a LTQ-Orbitrap XL or a LTQ-Orbitrap Velos mass spectrometer (Thermo Fisher Scientific) equipped with a nanoelectrospray source (Proxeon). Chromatographic separation was performed with in-house packed fused silica emitter with an inner diameter of 75 $\mu$m. Columns were packed with $C_{18}$-AQ RepoSil-Pur (3 $\mu$m, Dr. Maisch GmbH). Peptide separation was performed with a linear gradient of 5-30% Acetonitril with 0.5% acetic acid for 150 min at a flow rate of 200nl/min. After eluting from C18 column, peptides were ionized by electrospray ionization and transferred into the mass spectrometer. Full survey scan spectra (m/z = 300 - 1650) were acquired in the Orbitrap with a resolution of r = 60,000 after accumulation of 1,000,000 ions. The five most intense peaks from full MS scan in the LTQ-Orbitrap were isolated (target value of 5,000) and fragmented in the linear ion trap using CID (35% normalized collision energy). For LTQ-Orbitrap Velos measurements the 15 most intense peaks were selected for fragmentation in the linear iontrap.

The Raw data were analyzed by the MaxQuant software package (version 1.0.14.1.) as described in previously (Cox, J. et al. (2009). A practical guide to the MaxQuant computational platform for SILAC-based quantitative proteomics. Nature protocols 4, 698-705). Database search was performed with the Mascot search engine (version 2.2 Matrix Science) against a decoy zebrafish database (IPI version 3.54).

SILAC peptide pairs were detected and quantified by the Quant module using following parameters: Lys-C as digesting enzyme with a maximum of 2 missed cleavages, carbamidomethylation of cysteins as fixed modification, oxidation of methionine and acetylation of the protein N-terminus as variable modifications, SILAC amino acid labeling: Lys-6. In case of Lys-6 incorporation rate measurements no missed cleavage were allowed. Maximum mass deviation was set to 7 ppm for the peptide mass and 0.5 Da for MS/MS ions. For identification of peptides and proteins a false discovery rate (FDR) of 1% were used and only peptides with minimum of six amino acid length were considered for identification. For SILAC analysis, two ratio counts were set as a minimum for quantification.

## Example 5: Gene Ontology analysis

Differential protein abundance was checked between the developmental stages 72 and 120 hpf in respect to functional annotation based on Gene Ontology (GO). GO annotation was based on the UniProtKB GOA available at http://www.ebi.ac.uk/GOA. The most representative cellular compartment terms were chosen for hieriachical cluster analysis and heatmap plotting using the MultiExperiment Viewer CITE (Saeed AI, Bhagabati NK, Braisted JC, Liang W, Sharov V, Howe EA, et al. TM4 microarray software suite. Methods in Enzymology. 2006;411:134-93.). The mean log2 SILAC ratio of the proteins associated with each GO term was calculated to compare the abundance relative to the heavy standard between 120 and 72 hpf.

## Example 6: Dissection of adult zebrafish organs and preparation of embryonic zebrafish hearts

The zebrafish is an excellent model system for tissue regeneration. This process was analyzed by the incorporation of Lys-6 into regenerating fins. Therefore the upper lobe of the fin was amputated and the re-growing tissue after 1 week collected. For the control experiment the uninjured fin from the same animal was used respectively (Fig. 5c). By GeLC-MS analysis more than 2600 zebrafish proteins could be identified and an increase from 3% in regular wild type fins to 10% Lys-6 incorporation in regenerating fins was observed **(Fig. 1c).** This rather low increase during regeneration indicates that the proportion of labeled versus unlabeled amino acids in the whole fish is still low after this short term labeling and therefore newly synthesised proteins are mainly recycled from degraded proteins (Doherty, M.K. et al. (2005) Proteome dynamics in complex organisms: using stable isotopes to monitor individual protein turnover rates. Proteomics 5, 522-533)

To compare both incorporation rates more in detail both ratios to each other and labeled proteins with similar biological functions and localizations were plotted **(Fig. ld).** Not surprisingly circulating blood/serum proteins derived from other parts of the body showed similar Lys-6 labeling rates.

In contrast the majority of proteins including collagens, histones, and ribosomal proteins were observed with higher Lys-6 incorporations rates in the regenerating tissue, reflecting newly created fin tissue. For several candidates no SILAC ratios could be observed in the uninjured control fins whereas the regenerating fins showed several newly synthesized SILAC peptides with labeling rates up to 35%.

For example, Actinodin 2 and 4 with a labeling rate of $\sim$ 13% (Fig.1d) were detected. Recently for this group of proteins it was shown that Actinodin 1 and 2 are essential candidates for fin development (Zhang, J. et al. Loss of fish actinotrichia proteins and the fin-to-limb transition. Nature 466, 234-237).

Taken together, the pulse SILAC labeling can be used to identify the rate of newly synthesized proteins during regenerative processes and the detection of several transcripton factors, deacetylases, and kinases shows that our method is sensitive enough to perform an in depth proteome analysis.

Next, Lys-6 administration was prolonged for several months to monitor the long term effects of the diet and to measure the label efficiency. An expansion of SILAC labeling up to 11 month results in a relatively high incorporation of up to 85% in average **(Fig. 6a)**.

However, to achieve precise SILAC protein quantitation we seek to obtain complete labeled zebrafish. Therefore the F1 generation was bred to increase the Lys-6 incorporation to more than 95%, which is necessary for proper SILAC quantitation. For the F1 generation a reduced number of offsprings (~50% viable eggs) and a slight delay of growth **(Fig. 11a)** was observed. This is probably due to differences in the composition of the SILAC diet compared to the regular diet which shows egg survival rates between 60-80% (Siccardi, A.J., 3rd et al. (2009) Growth and survival of zebrafish (Danio rerio) fed different commercial and laboratory diets. Zebrafish 6, 275-280).

The analysis of one week old larvae revealed an average labeling of ~90%. One of the most abundant proteins the yolk precursor protein vitellogenin which is synthesised in the maternal liver was observed with a SILAC labeling of 95%. Other candidates with a low turnover such as nuclear histone proteins showed similar high SILAC ratios.

Hence, an almost complete labeling was achieved during the growth of the F1 and all subsequent generations.

However, to increase the survival rate during early developmental stages it was necessary to supplement the SILAC diet once per week with a regular non-labeled fish diet for time period of 6 weeks. After this critical period the heavy diet was used. Although a slight reduction of the initial labeling rate was observed, a complete labeling could be measured after 5 month **(Fig. 11c; Fig. 6a)**

As an example for a fully labeled tissue, two Lys-6 labeled SILAC peptides from the adult heart tissue of the F1 generation is shown in Figure 6b. The corresponding natural peptide has a much lower intensity and mixing equal amounts of non-labeled heart tissue results in a 1:1 SILAC ratio of these candidates. Similar, other organs such as brain and gills showed also high incorporation. An example for a low turnover protein is shown for the SILAC-pair from the Crystallin protein of the F2 generation **(Fig. 11b)**.

To demonstrate the accuracy of SILAC based quantification in fish, a mixture of heart tissue from labeled ($H_{SILAC}$) and unlabeled fish ($H_{Light\ 1a\ -\ 1c}$) was measured in technical triplicate experiments. By in solution digestions ~300 proteins with at least 2 peptides in all three runs were quantified.

Because SILAC tissues were used as an internal standard the ratios of ratios were calculated as shown in **(Fig. 2a)**. The direct comparison of $log_2$-SILAC ratios between replicates revealed a straight line with Pearson correlation r ranges from 0.93 to 0.96 and a slope range from 0.98 to 1.0 is in agreement with the 1:1 mix between labeled and unlabeled proteins.

Next, labeled heart protein lysate was spiked into three unlabeled hearts ($H_{Light1-3}$) to investigate the biological variation between different individuals. **Fig. 2b** shows similar statistical values with *r* ranging from 0.61 to 0.85 and with a slope range from 1.03 to 1.05. Calculating the mean ratio between unlabeled hearts revealed that only 2 candidates out of ~300 were detected with a two-fold change. Thus, this exemplary SILAC quantitation in zebrafish demonstrates the same statistical accuracy as proven for cell culture and other living animals.

To further validate the method embryonic zebrafish hearts at 72 h and 120 h after fertilization (hpf) were compared. A zebrafish strain was used, which expresses the green fluorescence protein (GFP) under the control of the cardiac myosin light chain 2 promoter for specific isolation of early embryonic heart tissue (Huang, C.J., et al. (2003). Germ-line transmission of a myocardium-specific GFP transgene reveals critical regulatory elements in the cardiac myosin light chain 2 promoter of zebrafish. Dev Dyn 228, 30-40; D'Amico, L., Scott, I.C., Jungblut, B. & Stainier, D.Y. (2007) A mutation in zebrafish hmgcrlb reveals a role for isoprenoids in vertebrate heart-tube formation. Curr Biol 17, 252-259. **(Fig. 7a)**.

For the analysis ~400 GFP positive hearts were isolated, which yielded in approx. 20 $\mu$g proteins from both developmental stages, respectively. As a labeled protein standard total protein lysates from 72 hpf embryos were used and equal protein amounts were mixed with unlabeled heart tissue from both developmental stages **(Fig. 7b)**. After 1D-Gelectrophoresis and in-gel digestion samples we quantified 1398 proteins between both time points with a false discovery rate lower than 1%.

In addition, for 172 proteins a fold change $\geq 2$ after 120 hpf compared to 72 hpf was observed and a GO-term analysis of those regulated candidates in relation to all identified proteins revealed a clear overrepresentation of GO-terms belonging to plasma membrane, sarcomeric structures and calcium signaling. Selected SILAC pairs of PCNA (proliferating cell nuclear antigen), Lactate dehydrogenase, and Nebulin were shown in **(Fig. 7d)**.

This finding is consistent with a progressive differentiation and maturation of subcellular structures like the contractile apparatus and the sarcoplasmatic reticulum during heart development **(Fig. 7e)**.

These results demonstrates that the SILAC Zebrafish which was labeled using the method according to the present invention allows large scale quantitative analysis of early zebrafish embryonic development.

**Claims**

1. A method for metabolic labeling of a target organism with a stable isotope, wherein the stable isotope is transferred from a label source to the target organism

   a) via one or more food chains, each food chain comprising of one or more cascades of ancestor organisms, wherein each cascade forms the ancestor organism for a successor organism of the following cascade, and wherein at least one food chain comprises

   i) a straight food chain comprising at least two cascades, wherein at least two ancestor organisms of the at least two cascades are connected in series; or
   ii) a branched food chain, wherein at least two ancestor organisms of the same or of different cascades are arranged in parallel; or

   b) via one or more one food chains of a) in combination with at least one synthetic diet labeled with the stable isotope as direct label source;

   wherein the target organism is fish;
   wherein the target organism is fed with a diet composition comprising material, cells and/or tissues derived from one or more labeled ancestor organisms of said one or more food chains or a combination thereof; wherein optionally the diet composition further comprises at least one synthetic diet labeled with the stable isotope; and wherein the stable isotope used is $^{13}C$, $^{15}N$, and/or $^2H$, or a combination thereof.

2. The method according to claim 1, wherein the label source is one or more labeled amino acids, and/or wherein optionally the labeled amino acid is lysine and/or arginine, or wherein optionally the labeled amino acid is $^{13}C$-labeled lysine.

3. The method according to any one of claim 1, wherein the label source is a labeled amino acid mixture or a labeled protein hydrolysate.

4. The method according to any one of claims 1 to 3, wherein the enrichment of said stable isotope in said target organism is at least 70% and/or, wherein optionally the target organism is a fish of the class *Actinopterygii,* optionally of the order *Cypriniformes* or the order *Beloniformes,* and/or wherein optionally the target organism is a fish of the family *Cyprinidae* or the family *Adrianichthyidae,* and/or wherein optionally the target organism is a fish of the genus *Danio* or the genus *Oryziinae,* and/or wherein optionally the target organism is a fish of the species *Oryzias latipes* or the species *Danio rerio,* and/or wherein preferably the target organism is *Danio rerio.*

5. The method according to any one of claims 1 to 4, wherein the ancestor organism of said one or more food chains is a prokaryotic or eukaryotic organism such as an animal, a plant or a fungus, wherein optionally the prokaryotic organism is a cyanobacterium, optionally the cyanobacterium being selected from the group consisting of *Synecococcus* and *Spirulina,* or wherein optionally the eukaryotic organism is a unicellular or multi-cellular plant cell, optionally the plant cell being an algae selected from the group consisting of Chlamydomonas, Scenedesmus and Chlorella.

6. The method according to any one of claims 1 to 5, wherein the direct ancestor organism of said at least one food chain used for transferring the label to said target organism has an average SILAC label of at least 40%, or wherein optionally the direct ancestor organism of said at least one food chain used for transferring the label to said target organism has an average global uniform label of at least 40%.

7. A method of producing a diet for metabolic labeling of a target organism with a stable isotope, comprising

   i) producing one or more ancestor organisms of a straight food chain comprising at least two cascades, wherein the one or more ancestor organisms are labeled with a stable isotope, and wherein at least two labeled ancestor organisms are connected in series, and wherein at least one labeled ancestor organism is an organism of at least the second or higher cascade; and/or

ii) producing at least two ancestor organisms of a branched food chain, wherein the at least two ancestor organisms are each labeled with a stable isotope and wherein the at least two labeled ancestor organisms of the same or of different cascades are arranged in parallel; and

iii) processing said one or more labeled ancestor organisms obtained in i) and/or ii) to obtain a diet for feeding the target organism, wherein optionally the metabolic labeling is an amino acid specific labeling, and/or wherein optionally the ancestor organism of the lowest cascade is an auxotroph;

wherein the target organism is fish;

wherein the target organism is fed with a diet composition comprising material, cells and/or tissues derived from one or more labeled ancestor organisms of said one or more food chains or a combination thereof; wherein optionally the diet composition further comprises at least one synthetic diet labeled with the stable isotope; and wherein the stable isotope used is $^{13}$C, $^{15}$N, and/or $^{2}$H, or a combination thereof.

8. The method according to claim 7, wherein the metabolic labeling is a global uniform labeling.

9. The method according to any one of claims 7 to 8, further comprising adding a synthetic diet comprising the stable isotope label as an additional label source, wherein optionally the diet comprises cells and/or tissues derived from one or more labeled ancestor organisms of said one or more food chains, and/or wherein optionally the diet comprises a combination of two or more ancestor organisms of different cascades, and/or wherein optionally the ancestor organism of the lowest cascade is fuelled with isotope label using a synthetic medium, and/or wherein optionally the target organism is *D. rerio* (Zebrafish) or an economically important fish.

10. A diet composition for metabolic labeling of a target organism with a stable isotope, wherein the diet composition comprises material, cells, and/or tissues of

i) at least one ancestor organism of at least the second cascade of a straight food chain comprising at least two cascades, wherein the at least one ancestor organism is labeled with a stable isotope and wherein at least two labeled ancestor organisms are connected in series; and/or

ii) at least two ancestor organisms of a branched food chain, wherein the at least two ancestor organisms are each labeled with a stable isotope and wherein said at least two labeled ancestor organisms of the same or of different cascades are arranged in parallel

wherein the ancestor organism serving as feed for the target organism and has an average SILAC label of at least 40%, or wherein the ancestor organism serving as feed for the target organism has an average global uniform label of at least 40%; wherein the target organism is fish;

wherein the target organism is fed with a diet composition comprising material, cells and/or tissues derived from one or more labeled ancestor organisms of said one or more food chains or a combination thereof; wherein optionally the diet composition further comprises at least one synthetic diet labeled with the stable isotope; and wherein the stable isotope used is $^{13}$C, $^{15}$N, and/or $^{2}$H, or a combination thereof.

11. Use of the diet composition according to claim 10 for producing a metabolically labeled fish target organism as a reference for a quantitative proteomic approach, or optionally for pulse SILAC labeling.

12. Use of a fish target organism labeled by the method according to any one of claims 1 to 6 as a spike-in standard in quantitative proteomics approaches.

**Patentansprüche**

1. Verfahren zur metabolischen Markierung eines Zielorganismus mit einem stabilen Isotop, wobei das stabile Isotop von einer Markierungsquelle auf den Zielorganismus übertragen wird

a) über eine oder mehrere Nahrungsketten, wobei jede Nahrungskette eine oder mehreren Kaskaden von Vorläuferorganismen umfasst, wobei jede Kaskade den Vorläuferganismus für einen Nachfolgeorganismus der folgenden Kaskade bildet und wobei mindestens eine Nahrungskette umfasst

i) eine gerade Nahrungskette, die mindestens zwei Kaskaden umfasst, wobei mindestens zwei Vorläuferorganismen der mindestens zwei Kaskaden in Reihe angeordnet sind; oder

ii) eine verzweigte Nahrungskette, wobei mindestens zwei Vorläuferorganismen derselben oder verschiedener Kaskaden parallel angeordnet sind; oder

b) über eine oder mehrere Nahrungsketten von a) in Kombination mit mindestens einer synthetischen Nahrung, die mit dem stabilen Isotop als direkte Markierungsquelle markiert ist;

wobei der Zielorganismus Fisch ist;
wobei der Zielorganismus mit einer Nahrungszusammensetzung gefüttert wird, die Material, Zellen und/oder Gewebe umfasst, welche von einem oder mehreren markierten Vorläuferorganismen der einen oder mehreren Nahrungsketten oder einer Kombination davon stammen; wobei optional die Nahrungszusammensetzung ferner mindestens eine synthetische Nahrung umfasst, die mit dem stabilen Isotop markiert ist; und wobei das verwendete stabile Isotop $^{13}$C, $^{15}$N und/oder $^{2}$H oder eine Kombination davon ist.

2. Verfahren nach Anspruch 1, wobei die Markierungsquelle eine oder mehrere markierte Aminosäuren ist und/oder wobei optional die markierte Aminosäure Lysin und/oder Arginin ist oder wobei die markierte Aminosäure optional $^{13}$C-markiertes Lysin ist.

3. Verfahren nach Anspruch 1, wobei die Markierungsquelle eine markierte Aminosäuremischung oder ein markiertes Proteinhydrolysat ist.

4. Verfahren nach einem der Ansprüche 1 bis 3, wobei die Anreicherung des stabilen Isotops im Zielorganismus mindestens 70% beträgt und/oder optional der Zielorganismus ein Fisch der Klasse *Actinopterygii,* optional der Ordnung *Cypriniformes* oder der Ordnung *Beloniformes* ist, und/oder wobei optional der Zielorganismus ein Fisch der Familie *Cyprinidae* oder der Familie *Adrianichthyidae* ist, und/oder wobei optional der Zielorganismus ein Fisch der Gattung *Danio* oder der Gattung *Oryziinae* ist, und/oder wobei der Zielorganismus optional ein Fisch der Art *Oryzias latipes* oder der Art *Danio rerio* ist und/oder wobei vorzugsweise der Zielorganismus *Danio rerio* ist.

5. Verfahren nach einem der Ansprüche 1 bis 4, wobei der Vorläuferorganismus der einen oder mehreren Nahrungsketten ein prokaryotischer oder eukaryotischer Organismus wie zum Beispiel ein Tier, eine Pflanze oder ein Pilz ist, wobei optional der prokaryotische Organismus ein Cyanobakterium ist, wobei das Cyanobakterium optional ausgewählt ist aus der Gruppe bestehend aus *Synecococcus* und *Spirulina,* oder wobei optional der eukaryotische Organismus eine einzellige oder mehrzellige Pflanzenzelle ist, wobei die Pflanzenzelle optional eine Alge ausgewählt aus der Gruppe bestehend aus Chlamydomonas, Scenedesmus und Chlorella ist.

6. Verfahren nach einem der Ansprüche 1 bis 5, wobei der direkte Vorläuferorganismus der mindestens einen Nahrungskette, der zur Übertragung der Markierung auf den Zielorganismus verwendet wird, eine durchschnittliche SILAC-Markierung von mindestens 40% aufweist, oder wobei optional der direkte Vorläuferorganismus der mindestens einen Nahrungskette, der zur Übertragung der Markierung auf den Zielorganismus verwendet wird, eine durchschnittliche globale einheitliche Markierung von mindestens 40% aufweist.

7. Verfahren zur Herstellung einer Nahrung zur metabolischen Markierung eines Zielorganismus mit einem stabilen Isotop, umfassend

i) Herstellen eines oder mehrerer Vorläuferorganismen einer geraden Nahrungskette umfassend mindestens zwei Kaskaden, wobei der oder die Vorläuferorganismen mit einem stabilen Isotop markiert sind, und wobei mindestens zwei markierte Vorläuferorganismen in Reihe angeordnet sind, und wobei mindestens ein markierter Vorläuferorganismus ein Organismus von mindestens der zweiten oder höheren Kaskade ist; und/oder
ii) Herstellen von mindestens zwei Vorläuferorganismen einer verzweigten Nahrungskette, wobei die mindestens zwei Vorläuferorganismen jeweils mit einem stabilen Isotop markiert sind und wobei die mindestens zwei markierten Vorläuferorganismen derselben oder verschiedener Kaskaden parallel angeordnet sind; und
iii) Verarbeiten des einen oder der mehreren markierten Vorläuferorganismen, die in i) und/oder ii) erhalten wurden, um eine Nahrung zum Füttern des Zielorganismus zu erhalten, wobei optional die metabolische Markierung eine aminosäurespezifische Markierung ist und/oder wobei optional der Vorläuferorganismus der untersten Kaskade auxotroph ist;

wobei der Zielorganismus Fisch ist;
wobei der Zielorganismus mit einer Nahrungszusammensetzung gefüttert wird, die Material, Zellen und/oder Gewebe umfasst, welche von einem oder mehreren markierten Vorläuferorganismen der einen oder mehreren Nahrungsketten oder einer Kombination davon stammen; wobei optional die Nahrungszusammensetzung ferner mindestens eine synthetische Nahrung umfasst, die mit dem stabilen Isotop markiert ist; und wobei das verwendete stabile Isotop $^{13}$C, $^{15}$N und/oder $^{2}$H oder eine Kombination davon ist.

**8.** Verfahren nach Anspruch 7, wobei die metabolische Markierung eine globale einheitliche Markierung ist.

**9.** Verfahren nach einem der Ansprüche 7 bis 8, ferner umfassend die Zugabe einer synthetischen Nahrung umfassend die Markierung mit einem stabilen Isotop als eine zusätzliche Markierungsquelle, wobei die Nahrung optional Zellen und/oder Gewebe umfasst, welche von einem oder mehreren markierten Vorläuferorganismen der einen oder mehreren Nahrungsketten stammen, und/oder wobei die Nahrung optional eine Kombination von zwei oder mehreren Vorläuferorganismen unterschiedlicher Kaskaden umfasst, und/oder wobei optional der Vorläuferorganismus der untersten Kaskade mit Isotopenmarkierung unter Verwendung eines synthetischen Mediums markiert wird, und/oder wobei optional der Zielorganismus *D. rerio* (Zebrafisch) oder ein wirtschaftlich wichtiger Fisch ist.

**10.** Nahrungszusammensetzung zur metabolischen Markierung eines Zielorganismus mit einem stabilen Isotop, wobei die Nahrungszusammensetzung Material, Zellen und/oder Gewebe von

i) mindestens einem Vorläuferorganismus von mindestens der zweiten Kaskade einer geraden Nahrungskette umfassend mindestens zwei Kaskaden umfasst, wobei der mindestens eine Vorläuferorganismus mit einem stabilen Isotop markiert ist und wobei mindestens zwei markierte Vorläuferorganismen in Reihe angeordnet sind; und/oder
ii) mindestens zwei Vorläuferorganismen einer verzweigten Nahrungskette umfasst, wobei die mindestens zwei Vorläuferorganismen jeweils mit einem stabilen Isotop markiert sind und wobei die mindestens zwei markierten Vorläuferorganismen derselben oder verschiedener Kaskaden parallel angeordnet sind,

wobei der Vorläuferorganismus als Futtermittel für den Zielorganismus dient und eine durchschnittliche SILAC-Markierung von mindestens 40% aufweist, oder wobei der Vorläuferorganismus, der als Futtermittel für den Zielorganismus dient, eine durchschnittliche globale einheitliche Markierung von mindestens 40% aufweist; wobei der Zielorganismus Fisch ist; wobei der Zielorganismus mit einer Nahrungszusammensetzung gefüttert wird, die Material, Zellen und/oder Gewebe umfasst, welche von einem oder mehreren markierten Vorläuferorganismen der einen oder mehreren Nahrungsketten oder einer Kombination davon stammen; wobei optional die Nahrungszusammensetzung ferner mindestens eine synthetische Nahrung umfasst, die mit dem stabilen Isotop markiert ist; und wobei das verwendete stabile Isotop $^{13}$C, $^{15}$N und/oder $^{2}$H oder eine Kombination davon ist.

**11.** Verwendung der Nahrungszusammensetzung nach Anspruch 10 zur Herstellung eines metabolisch markierten Fisch-Zielorganismus als Referenz für einen quantitativen proteomischen Ansatz oder optional zur Puls-SILAC-Markierung.

**12.** Verwendung eines mit dem Verfahren nach einem der Ansprüche 1 bis 6 markierten Fisch-Zielorganismus als Spitzenstandard in quantitativen Proteomik-Ansätzen.

**Revendications**

**1.** Procédé de marquage métabolique d'un organisme cible par un isotope stable, sachant que l'isotope stable est transféré d'une source de marqueur à l'organisme cible

a) via une ou plusieurs chaînes alimentaires, chaque chaîne alimentaire étant composée d'une ou de plusieurs cascades d'organismes ascendants, sachant que chaque cascade forme l'organisme ascendant pour un organisme descendant de la cascade suivante, et sachant qu'au moins une chaîne alimentaire comprend

i) une chaîne alimentaire droite comprenant au moins deux cascades, sachant qu'au moins deux organismes ascendants des au moins deux cascades sont connectés en série ; ou
ii) une chaîne alimentaire ramifiée, sachant qu'au moins deux organismes ascendants de la même cascade ou de cascades différentes sont agencés en parallèle ; ou

b) via une ou plusieurs chaînes alimentaires de a) en combinaison avec au moins un régime synthétique marqué par l'isotope stable comme source de marqueur directe ;

sachant que l'organisme cible est du poisson ;
sachant que l'organisme cible est alimenté avec une composition de régime comprenant de la matière, des cellules et/ou des tissus dérivés d'un ou de plusieurs organismes ascendants marqués de ladite une ou desdites plusieurs chaînes alimentaires ou une combinaison de ceux-ci ; sachant que facultativement la composition de régime comprend en outre au moins un régime synthétique marqué par l'isotope stable ; et
sachant que l'isotope stable utilisé est $^{13}$C, $^{15}$N, et/ou $^2$H, ou une combinaison de ceux-ci.

2.  Le procédé selon la revendication 1, sachant que la source de marqueur est un ou plusieurs acides aminés marqués, et/ou sachant que facultativement l'acide aminé marqué est de la lysine et/ou de l'arginine, ou sachant que facultativement l'acide aminé marqué est de la lysine marquée par $^{13}$C.

3.  Le procédé selon la revendication 1, sachant que la source de marqueur est un mélange d'acides aminés marqué ou un hydrolysat protéique marqué.

4.  Le procédé selon l'une quelconque des revendications 1 à 3, sachant que l'enrichissement dudit isotope stable dans ledit organisme cible est d'au moins 70 % et/ou sachant que facultativement l'organisme cible est un poisson de la classe Actinopterygii, facultativement de l'ordre Cypriniformes ou de l'ordre Beloniformes, et/ou sachant que facultativement l'organisme cible est un poisson de la famille Cyprinidae ou de la famille Adrianichthyidae, et/ou sachant que facultativement l'organisme cible est un poisson du genre Danio ou du genre Oryziinae, et/ou sachant que facultativement l'organisme cible est un poisson de l'espèce Oryzias latipes ou de l'espèce Danio rerio, et/ou sachant que de préférence l'organisme cible est Danio rerio.

5.  Le procédé selon l'une quelconque des revendications 1 à 4, sachant que l'organisme ascendant de ladite une ou desdites plusieurs chaînes alimentaires est un organisme procaryote ou eucaryote tel qu'un animal, une plante ou un mycète, sachant que facultativement l'organisme procaryote est une cyanobactérie, facultativement la cyanobactérie étant sélectionnée dans le groupe constitué par Synecococcus et Spirulina, et sachant que facultativement l'organisme eucaryote est une cellule végétale unicellulaire ou multicellulaire, facultativement la cellule végétale étant une algue sélectionnée dans le groupe constitué par Chlamydomonas, Scenedesmus et Chlorella.

6.  Le procédé selon l'une quelconque des revendications 1 à 5, sachant que l'organisme ascendant direct de ladite au moins une chaîne alimentaire utilisée pour transférer le marqueur audit organisme cible présente un marqueur SILAC moyen d'au moins 40 %, ou sachant que facultativement l'organisme ascendant direct de ladite au moins une chaîne alimentaire utilisée pour transférer le marqueur audit organisme cible présente un marqueur uniforme global moyen d'au moins 40 %.

7.  Procédé de production d'un régime pour le marquage métabolique d'un organisme cible par un isotope stable, comprenant

    i) la production d'un ou de plusieurs organismes ascendants d'une chaîne alimentaire droite comprenant au moins deux cascades, sachant que l'un ou les plusieurs organismes ascendants sont marqués par un isotope stable, et sachant qu'au moins deux organismes ascendants marqués sont connectés en série, et sachant qu'au moins un organisme ascendant marqué est un organisme d'au moins la deuxième cascade ou une cascade supérieure ; et/ou
    ii) la production d'au moins deux organismes ascendants d'une chaîne alimentaire ramifiée, sachant que les au moins deux organismes ascendants sont chacun marqués par un isotope stable et sachant que les au moins deux organismes ascendants marqués de la même cascade ou de cascades différentes sont agencés en parallèle ; et
    iii) le traitement dudit un ou desdits plusieurs organismes ascendants marqués obtenus en i) et/ou ii) pour obtenir un régime destiné à alimenter l'organisme cible, sachant que facultativement le marquage métabolique est un marquage spécifique aux acides aminés, et/ou sachant que facultativement l'organisme ascendant de la cascade inférieure est un auxotrophe ;

    sachant que l'organisme cible est du poisson ;
    sachant que l'organisme cible est alimenté avec une composition de régime comprenant de la matière, des cellules et/ou des tissus dérivés d'un ou de plusieurs organismes ascendants marqués de ladite une ou desdites plusieurs chaînes alimentaires ou une combinaison de ceux-ci ; sachant que facultativement la composition de régime comprend en outre au moins un régime synthétique marqué par l'isotope stable ; et
sachant que l'isotope stable utilisé est $^{13}$C, $^{15}$N, et/ou $^{2}$H, ou une combinaison de ceux-ci.

8. Le procédé selon la revendication 7, sachant que le marquage métabolique est un marquage uniforme global.

9. Le procédé selon l'une quelconque des revendications 7 à 8, comprenant en outre l'ajout d'un régime synthétique comprenant le marqueur à isotope stable comme source de marqueur supplémentaire,
sachant que facultativement le régime comprend des cellules et/ou des tissus dérivés d'un ou de plusieurs organismes ascendants marqués de ladite une ou desdites plusieurs chaînes alimentaires, et/ou sachant que facultativement le régime comprend une combinaison de deux organismes ascendants ou plus de différentes cascades, et/ou sachant que facultativement l'organisme ascendant de la cascade inférieure est alimentée en marqueur isotope moyennant un milieu synthétique, et/ou sachant que facultativement l'organisme cible est D. rerio (dard-perche) ou un poisson économiquement important.

10. Composition de régime pour le marquage métabolique d'un organisme cible par un isotope stable, sachant que la composition de régime comprend de la matière, des cellules, et/ou des tissus de

i) au moins un organisme ascendant d'au moins la deuxième cascade d'une chaîne alimentaire droite comprenant au moins deux cascades, sachant que l'au moins un organisme ascendant est marqué par un isotope stable et sachant qu'au moins deux organismes ascendants marqués sont connectés en série ; et/ou
ii) au moins deux organismes ascendants d'une chaîne alimentaire ramifiée, sachant que les au moins deux organismes ascendants sont chacun marqués par un isotope stable et sachant que lesdits au moins deux organismes ascendants marqués de la même cascade ou de cascades différentes sont agencés en parallèle ;

sachant que l'organisme ascendant servant d'aliment pour l'organisme cible présente un marqueur SILAC moyen d'au moins 40 %, ou sachant que l'organisme ascendant servant d'aliment pour l'organisme cible présente un marqueur uniforme global moyen d'au moins 40 % ;
sachant que l'organisme cible est du poisson ;
sachant que l'organisme cible est alimenté avec une composition de régime comprenant de la matière, des cellules et/ou des tissus dérivés d'un ou de plusieurs organismes ascendants marqués de ladite une ou desdites plusieurs chaînes alimentaires ou une combinaison de ceux-ci ; sachant que facultativement la composition de régime comprend en outre au moins un régime synthétique marqué par l'isotope stable ; et
sachant que l'isotope stable utilisé est $^{13}$C, $^{15}$N, et/ou $^{2}$H, ou une combinaison de ceux-ci.

11. Utilisation de la composition de régime selon la revendication 10 pour la production d'un organisme cible étant du poisson métaboliquement marqué comme référence pour une approche protéomique quantitative, ou facultativement pour un marquage SILAC de brève durée.

12. Utilisation d'un organisme cible poisson marqué par le procédé selon l'une quelconque des revendications 1 à 6 comme standard de Spike-in dans les approches protéomiques quantitatives.

Figure 1A

**a**

Figure 1B

**b**

Figure 2A/B          **a**

**Straight and branched food chains**

Branched food chain                    Straight food chain

Target organism                        Target organism

Ancestor 2c          *Cascade 2*

Ancestor 1a    Ancestor 1b

Ancestor 1c          *Cascade 1*

Label          Label                   Label

**b**

**Exemplary food chain**

Target organism

Ancestor 2a    Ancestor 2b          Ancestor 2c          Ancestor 2d    *Cascade 2*

Ancestor 1a    Ancestor 1b          Ancestor 1c          Ancestor 1d    *Cascade 1*

Label          Label                 Label          Label

Figure 3A    **a**

Figure 3B        **b**

Figure 4A          a

Figure 4B

b

Figure 5A/B

Figure 5C/D

Figure 6A/B

Figure 7A-C

Figure 7D

Figure 7E

**e**

Figure 8A-D

a

b

adult SILAC diet

larvaL SILAC diet

c

d

Figure 9A

Figure 9B **b**

Figure 10

Beta B3-Crystallin in the eye, VGSIIVESGPWVGFEQK

Apolipoprotein A-I in the liver, AFESNIEETK

Figure 11 A-C

Figure 12

**Heart**

average SILAC label: 92%

Actin, cytoplasmic 2

**Brain**

average SILAC label: 93%

Calbindin 2

**Gills**

average SILAC label: 93%

Na+/K+ ATPase alpha subunit

**Muscle**

average SILAC label: 94%

Muscle-specific creatine kinase

Figure 13A

Figure 13B

**b**

Figure 13C

**c**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2000012140 A

### Non-patent literature cited in the description

- **SCHOENHEIMER, R. ; RITTENBERG, D.** Deuterium as an Indicator in the Study of Intermediary Metabolism. *Science,* 1935, vol. 82, 156-157
- **WATERLOW, J.C.** Total protein turnover in animals and man. *Nutr Rev,* 1970, vol. 28, 115-118
- **ONG ; MANN.** Stable isotope labeling by amino acids in cell culture, SILAC, as a simple and accurate approach to expression proteomics. *Mol Cell Proteomics,* 2002, vol. 1, 376-386
- **WESTMAN-BRINKMALM, A. et al.** SILAC zebrafish for quantitative analysis of protein turnover and tissue regeneration. *J Proteomics,* 2011, vol. 75, 425-434
- **ONG, S.E. ; MANN, M.** Stable isotope labeling by amino acids in cell culture for quantitative proteomics. *Methods Mol Biol,* 2007, vol. 359, 37-52
- **SOUFI, B. et al.** Stable isotope labeling by amino acids in cell culture (SILAC) applied to quantitative proteomics of Bacillus subtilis. *J Proteome Res,* vol. 9, 3638-3646
- **GRUHLER, A. et al.** Quantitative phosphoproteomics applied to the yeast pheromone signaling pathway. *Mol Cell Proteomics,* vol. 4, 310-327
- **TOPS, B.B. et al.** Worms from Venus and Mars: proteomics profiling of sexual differences in Caenorhabditis elegans using in vivo 15 N isotope labeling. *J Proteome Res.,* 2010, vol. 9, 341.51
- **SURY, M.D. et al.** The SILAC fly allows for accurate protein quantification in vivo. *Mol Cell Proteomics,* vol. 9, 2173-2183
- **MCCLATCHY, D.B. et al.** 15N metabolic labeling of mammalian tissue with slow protein turnover. *J Proteome Res,* 2007, vol. 6, 2005-2010
- **WU, C.C.** Metabolic labeling of mammalian organism with stable isotoptes for quantitative proteomic analysis. *Anal Chem,* 2004, vol. 76, 2951-59
- **WU ; MACCOSS.** Quantitative proteomic analysis of mammalian organisms using metabolically labeled tissues. *Methods Mol Biol.,* 2007, vol. 359, 191-201

- **D. B. MCCLATCHY et al.** N metabolic labeling of mammalian tissue with slow protein turnover. *Journal of Proteome Research,* 2007, vol. 6 (5), 2005-2010
- **HUANG, C.J. et al.** Germ-line transmission of a myocardium-specific GFP transgene reveals critical regulatory elements in the cardiac myosin light chain 2 promoter of zebrafish. *Dev Dyn,* 2003, vol. 228, 30-40
- **BICHO, C.C. et al.** A genetic engineering solution to the "arginine conversion problem" in stable isotope labeling by amino acids in cell culture (SILAC). *Mol Cell Proteomics,* vol. 9, 1567-1577
- identifying and quantifying sites of protein methylation by heavy methyl SILAC. **ONG ; MANN.** Cur Protc Protein Sci. December 2006
- **RAPPSILBER, J. ; ISHIHAMA, Y. ; MANN, M.** Stop and go extraction tips for matrix-assisted laser desorption/ionization, nanoelectrospray, and LC/MS sample pretreatment in proteomics. *Anal Chem,* 2003, vol. 75, 663-670
- **SHEVCHENKO, A. et al.** In-gel digestion for mass spectrometric characterization of proteins and proteomes. *Nature protocols,* 2006, vol. 1, 2856-2860
- **WISNIEWSKI, J et al.** Combination of FASP and StageTip-based fractionation allows in-depth analysis of the hippocampal membrane proteome. *J Proteome Res,* 2009, vol. 8, 5674-5678
- **COX, J. et al.** A practical guide to the MaxQuant computational platform for SILAC-based quantitative proteomics. *Nature protocols,* 2009, vol. 4, 698-705
- **SAEED AI ; BHAGABATI NK ; BRAISTED JC ; LIANG W ; SHAROV V ; HOWE EA et al.** TM4 microarray software suite. *Methods in Enzymology,* 2006, vol. 411, 134-93
- **DOHERTY, M.K. et al.** Proteome dynamics in complex organisms: using stable isotopes to monitor individual protein turnover rates. *Proteomics,* 2005, vol. 5, 522-533
- **ZHANG, J. et al.** Loss of fish actinotrichia proteins and the fin-to-limb transition. *Nature,* vol. 466, 234-237

• **SICCARDI, A.J., 3RD et al.** Growth and survival of zebrafish (Danio rerio) fed different commercial and laboratory diets. *Zebrafish,* 2009, vol. 6, 275-280

• **D'AMICO, L. ; SCOTT, I.C. ; JUNGBLUT, B. ; STAINIER, D.Y.** A mutation in zebrafish hmgcrlb reveals a role for isoprenoids in vertebrate heart-tube formation. *Curr Biol,* 2007, vol. 17, 252-259